(19) Europäisches Patentamt / European Patent Office / Office européen des brevets

(11) **EP 2 969 369 B1**

## (12) EUROPEAN PATENT SPECIFICATION

(45) Date of publication and mention
of the grant of the patent:
**09.03.2022 Bulletin 2022/10**

(21) Application number: **14723550.1**

(22) Date of filing: **13.03.2014**

(51) International Patent Classification (IPC):
**B23K 26/06** (2014.01)   **A61B 18/26** (2006.01)
**A61B 18/20** (2006.01)   **A61N 5/067** (2006.01)

(52) Cooperative Patent Classification (CPC):
**A61B 18/26; B23K 26/0624;** A61B 18/203;
A61B 2018/00458; A61B 2018/00577;
A61B 2018/263

(86) International application number:
**PCT/US2014/026335**

(87) International publication number:
**WO 2014/160331 (02.10.2014 Gazette 2014/40)**

(54) **CONTROLLED PHOTOMECHANICAL AND PHOTOTHERMAL TISSUE TREATMENT IN THE PICOSECOND REGIME**

GESTEUERTE PHOTOMECHANISCHE UND PHOTOTHERMALE GEWEBEBEHANDLUNG IM PICOSEKUNDENBEREICH

TRAITEMENT PHOTOMÉCANIQUE ET PHOTOTHERMIQUE COMMANDÉ DE TISSU EN RÉGIME PICOSECONDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.03.2013   US 201361779411 P
27.11.2013   US 201361909563 P**

(43) Date of publication of application:
**20.01.2016 Bulletin 2016/03**

(73) Proprietor: **Cynosure, LLC
Westford, MA 01886 (US)**

(72) Inventors:
• **WELCHES, Richard, Shaun
Woburn, MA 01801 (US)**
• **MIRKOV, Mirko Georgiev
Chelmsford, MA 01824 (US)**

(74) Representative: **Purdylucey Intellectual Property
6-7 Harcourt Terrace
D02 FH73 Dublin 2 (IE)**

(56) References cited:
US-A1- 2002 125 230    US-A1- 2008 031 288
US-A1- 2011 313 408    US-B1- 6 482 199

**Description**

FIELD OF THE INVENTION

The present disclosure relates to an apparatus for delivering laser energy having a short pulse duration (e.g., less than about 1 nanosecond) and high energy output per pulse into tissues, resulting in tissue damage and tissue remodeling and regeneration.

BACKGROUND

Photothermal mechanisms for tissue treatment have been widely exploited for medical and cosmetic tissue treatments including dermatology treatments. Currently available light based (including laser) treatments for conditions such as scar modification rely on relatively aggressive thermal treatment. In order to achieve certain treatments at desired depths the level of photothermal temperature rise necessary as part of a desired treatment can result in unwanted/undesirable additional thermal damage to adjacent regions. In treating such medical and cosmetic conditions it is desirable to limit thermal damage to the target treatment area and avoid unnecessary thermal damage to areas outside the target treatment area. US Patent Publication No. US 2008/031288 discloses a picosecond laser apparatus and methods for its operation and use.

SUMMARY OF THE INVENTION

The disclosure relates to a system for tissue treatment, as set out in the appended claims.
The system includes a controller for controlling the pulse width to provide shock wave pressure emission intensity to the tissue adjacent the target at a shorter pulse width and a lesser shock wave pressure emission intensity to the tissue adjacent the target at a longer pulse width. In one embodiment, the controller controls the pulse width to provide a greater thermal effect at a relatively longer pulse width; this ensures a combination of thermal effect and mechanical effect in a tissue area and by apportioning them one can control the percentage of thermal effect damage relative to mechanical effect damage.
In another embodiment, the controller enables one pass of the laser at a shorter pulse width and at a relatively shallow depth and another pass at a relatively longer pulse width and a relatively deeper depth, alternatively, the controller enables one pass of the laser at a first depth and another pass of the laser at a second depth different from the first depth. In some embodiments, the controller enables a first pass of the laser and a second pass of the laser may only be fired during the electron ionization of the target. In another embodiment, the optical system has two or more foci for concentrating the laser emission to two or more adjacent targets at a depth in the tissue at a fluence sufficient to create electron ionization of the two or more adjacent targets.
In another aspect, the disclosure relates to a method for tissue treatment that includes providing a laser having a pulse width ranging from about 260 picoseconds to about 900 picoseconds, from about 300 picoseconds to about 775 picoseconds, from about 450 picoseconds to about 600 picoseconds, from about 260 picoseconds to about 500 picoseconds, or from about 260 picoseconds to about 300 picoseconds and a fluence ranging from about 0.8 J/cm$^2$ to about 50 J/cm$^2$, from about 0.8 J/cm$^2$ to about 25 J/cm$^2$, or from about from about 0.8 J/cm$^2$ to about 10 J/cm$^2$. The method also includes concentrating the laser emission to target at least one depth in the tissue at a fluence selected to exceed the electron ionization threshold of the target to result in an ablation volume of at least a portion of the target. The method includes controlling the pulse width to provide a pressure wave emission from the ablation volume to tissue adjacent the target.
In one embodiment, the laser has a wavelength of about 755 nm and the target is a blood cell. In another embodiment, the laser has a wavelength of about 1064 nm and the target is a depth of from about 1 mm to about 4 mm from the tissue surface. In some embodiments, concentrating the laser emission comprises concentrating the laser emission through at least one foci. In another embodiment, the method includes concentrating the laser emission to a depth of desired treatment. The method can include concentrating the laser emission to a depth of a region of injured tissue to be treated with pressure wave emission having a shock wave pressure intensity. The method can also include concentrating the laser emission to a depth of an organ to be treated with pressure wave emission having a shock wave pressure intensity.
Optionally, the at least one target is not at a depth and is rather at the surface of the skin tissue.

BRIEF DESCRIPTION OF THE FIGURES

Figure 1A, in a schematic diagram, illustrates an exemplary system having a wavelength-shifting resonator for generating picosecond pulses in accordance with various aspects of the applicants' teachings.

Figure 1B illustrates a tissue injury caused by a picosecond laser including an ablation volume of a cavitation bubble with a layer of tissue adjacent the cavitation bubble being subjected to relatively intense pressure and the next progressively outer layer(s) of tissue being subjected to relatively less intense pressure.

Figure 2(a) illustrates a parabolic lens cluster.

Figure 2(b) illustrates focusing the parabolic lens cluster shown in Figure 2(a) at an overlapping deep spot target area within the tissue.

Figure 3(a) illustrates two single lenses positioned at a skin surface each achieving a different focus distance and each achieving a different depth of penetration when exposed to the same wavelength and pulsewidth in the picosecond regime.

Figure 3(b) illustrates a quasi-parabolic quad cell micro lens array positioned at a skin surface and exposed to a wavelength at a pulsewidth in the picosecond regime, the quad cells micro lens array a single relatively deep spot treatment area.

Figure 3(c) illustrates a plurality of quasi-parabolic quad cell micro lens array clusters positioned at a skin surface and exposed to a wavelength at a pulsewidth in the picosecond regime, each cluster of the plurality targets a single relatively deep spot treatment area resulting in as many treatment areas as there are clusters.

Figure 3(d) illustrates the plurality of created in the injuries in the various spot treatment areas of Figure 3(c) with each injury including both an ablation lesion and a mechanically damaged region of tissue.

Figure 3(e) illustrates a plurality of single cell focus micro-lenses that create two distinct layers of spot treatment areas.

Figure 3(f) illustrates the two distinct layers of tissue injury created by the various spot treatment areas in Figure 3(e) with each injury including both an ablation lesion and a mechanically damaged region of tissue.

Figure 3(g) illustrates three layers of depth of tissue injury discussed in association with Figures 3(a)-3(e).

Figures 4(a)-4(c) illustrate a phased lens array approach that times the delivery of a plurality of injuries such that the mechanical injury (e.g., shockwave injury) is shaped to converge and/or focus on a single deeper target area.

Figures 5(a)-5(b) illustrate a sequential one two pulse arrangement for a picosecond drive pulse that is split by an adjustable beam splitter such that one split part can be delayed by an adjustable amount of time such that the delayed part arrives at the target while the target area is still ionized by the first non-delayed part and this can act to enable the second part to be immediately and fully absorbed by the target area and acts to drive a second pulse of expansion.

Figures 5(c)-5(d) illustrate that the point where the peak pressure provided by the first pulse is just beginning to wane (but is still in a plasma/ionized state) then consequently the initial shockwaves generated by the first pulse will detach (such that it is no longer driven by the ablation bubble expansion) and will begin to propagate into the tissue just as the second pulse arrives this can provide an enhanced lesion.

Figure 6 illustrates the generalized relationship between thermal energy and mechanical wave energy (e.g., shock wave and/or pressure wave energy) measured in psi as a function of pulse width in the picosecond and nanosecond regimes.

Figure 7(a) provides histology of tissue treated with a picosecond laser at 100x magnification.

Figure 7(b) provides histology of tissue treated with a picosecond laser at 400x magnification.

Figure 8(a) provides histology of tissue treated with a picosecond laser at 100x magnification.

Figure 8(b) provides histology of tissue treated with a picosecond laser at 400x magnification.

DETAILED DESCRIPTION

The present disclosure relates to laser systems having sub-nanosecond pulsing (e.g., picosecond pulsing). Exemplary systems are described in our U.S. Patents 7,929,579 7,5 86,957. US 2011/313408 and US 2008/031288. These patents disclose laser apparatuses and methods for their operation and use. Herein we describe certain improvements to such systems.

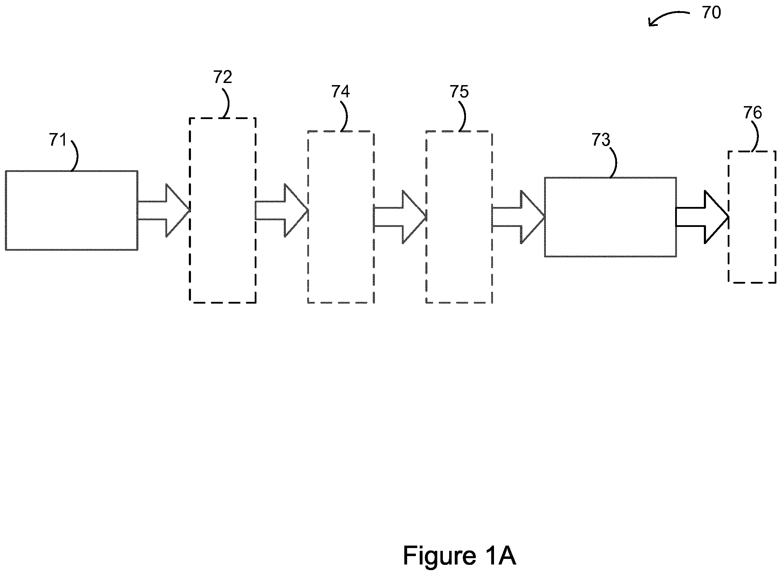

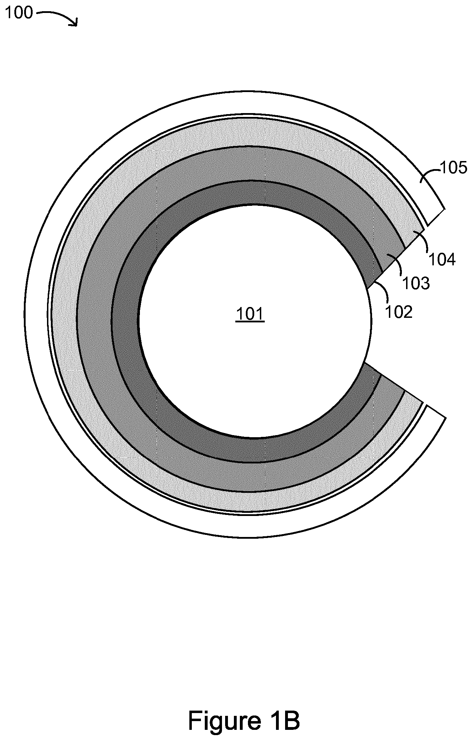

With reference now to FIG. 1A, an exemplary system 70 for the generation and delivery of picosecond-pulsed treatment radiation is schematically depicted. As shown in FIG. 1, the system generally includes a pump radiation source 71 for generating picosecond pulses at a first wavelength and a treatment beam delivery system 73 for delivering a pulsed treatment beam to the patient's skin.

The system optionally includes a wavelength-shifting resonator 72 for receiving the picosecond pulses generated by the pump radiation source 71 and emitting radiation at a second wavelength in response thereto to the treatment beam delivery system 73.

The pump radiation source 71 generally generates one or more pulses at a first wavelength to be transmitted to the wavelength-shifting resonator 72, and can have a variety of configurations. For example, the pulses generated by the pump radiation source 71 can have a variety of wavelengths, pulse durations, and energies. In some aspects, as will be discussed in detail below, the pump radiation source 71 can be selected to emit substantially monochromatic optical radiation having a wavelength that can be efficiently absorbed by the wavelength-shifting resonator 72 in a minimum number of passes through the gain medium. Additionally, it will be appreciated by a person skilled in the art in light of the present teachings that the pump radiation source 71 can be operated so as to generate pulses at various energies, depending for example, on the amount of energy required to stimulate emission by the wavelength-shifting resonator 72 and the amount of energy required to perform a particular treatment in light of the efficiency of the system 70 as a whole.

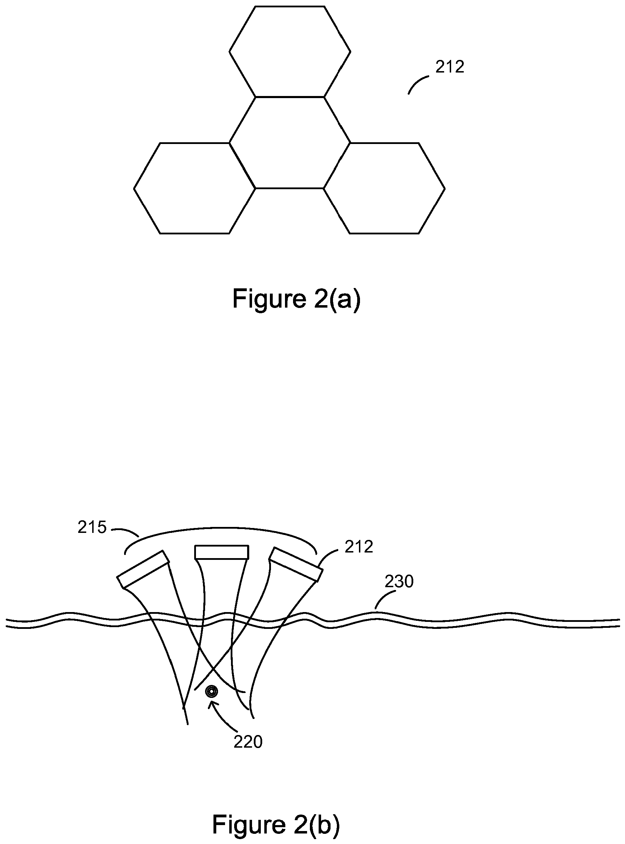

In various aspects, the pump radiation source 71 can be configured to generate picosecond pulses of optical radiation. That is, the pump radiation source can generate pulsed radiation exhibiting a pulse duration less than about 1000 picoseconds (e.g., within a range of about 500 picoseconds to about 800 picoseconds). In an exemplary embodiment, the pump radiation source 71 for generating the pump pulse at a first wavelength can include a resonator (or laser cavity containing a lasing medium), an electro-optical device (e.g., a Pockels cell), and a polarizer (e.g., a thin-film polarizer), as described for example with reference to FIG. 2 of U.S. Patent No. 7,586,957, issued on September 8, 2009 and entitled "Picosecond Laser Apparatus and Methods for Its Operation and Use".

In an exemplary embodiment, the lasing or gain medium of the pump radiation source 71 can be pumped by any conventional pumping device such as an optical pumping device (e.g., a flash lamp) or an electrical or injection pumping device. In an exemplary embodiment, the pump radiation source 71 comprises a solid state lasing medium and an optical pumping device. Exemplary solid state lasers include an alexandrite or a titanium doped sapphire (TIS) crystal, Nd:YAG lasers, Nd:YAP, Nd:YAlO$_3$ lasers, Nd:YAF lasers, and other rare earth and transition metal ion dopants (e.g., erbium, chromium, and titanium) and other crystal and glass media hosts (e.g., vanadate crystals such as YVO$_4$, fluoride glasses such as ZBLN, silica glasses, and other minerals such as ruby).

At opposite ends of the optical axis of the resonator can be first and second mirrors having substantially complete reflectivity such that a laser pulse traveling from the lasing medium towards second mirror will first pass through the polarizer, then the Pockels cell, reflect at second mirror, traverse Pockels cell a second time, and finally pass through polarizer a second time before returning to the gain medium. Depending upon the bias voltage applied to the Pockels cell, some portion (or rejected fraction) of the energy in the pulse will be rejected at the polarizer and exit the resonator along an output path to be transmitted to the wavelength-shifting resonator 72. Once the laser energy, oscillating in the resonator of the pump radiation source 71 under amplification conditions, has reached a desired or maximum amplitude, it can thereafter be extracted for transmission to the wavelength-shifting resonator 72 by changing the bias voltage to the Pockels cell such that the effective reflectivity of the second mirror is selected to output laser radiation having the desired pulse duration and energy output.

The wavelength-shifting resonator 72 can also have a variety of configurations in accordance with the applicant's present teachings, but is generally configured to receive the pulses generated by the pump radiation source 71 and emit radiation at a second wavelength in response thereto. In an exemplary embodiment, the wavelength-shifting resonator 72 comprises a lasing medium and a resonant cavity extending between an input end and an output end, wherein the lasing medium absorbs the pulses of optical energy received from the pump radiation source 71 and, through a process of stimulated emission, emits one or more pulses of optical laser radiation exhibiting a second wavelength.

As will be appreciated by a person skilled in the art in light of the present teachings, the lasing medium of the wavelength-shifting resonator can comprise a neodymium-doped crystal, including by way of non-limiting example solid state crystals of neodymium-doped yttrium-aluminum garnet (Nd:YAG), neodymium-doped pervoskite (Nd:YAP or Nd:YAlO$_3$), neodymium-doped yttrium-lithium-fluoride (Nd:YAF), and neodymium-doped vanadate (Nd:YVO$_4$) crystals. It will also be appreciated that other rare earth transition metal dopants (and in combination with other crystals and glass media hosts) can be used as the lasing medium in the wavelength-shifting resonator. Moreover, it will be appreciated that the solid state laser medium can be doped with various concentrations of the dopant so as to increase the absorption of the pump pulse within the lasing medium. By way of example, in some aspects the lasing medium can comprise between about 1 and about 3 percent neodymium.

The lasing medium of the wavelength-shifting resonator 72 can also have a variety of shapes (e.g., rods, slabs, cubes) but is generally long enough along the optical axis such that the lasing medium absorbs a substantial portion (e.g., most, greater than 80%, greater than 90%) of the pump pulse in two passes through the crystal. As such, it will be appreciated by a person skilled in the art that the wavelength of the pump pulse generated by the pump radiation source 71 and the absorption spectrum of the lasing medium of the resonator 72 can be matched to improve absorption. However, whereas prior art techniques tend to focus on maximizing absorption of the pump pulse by increasing crystal length, the resonator cavities disclosed herein instead utilize a short crystal length such that the roundtrip time of optical

$$t_{roundtrip} = 2\frac{n \cdot l_{resonator}}{c}$$

radiation in the resonant cavity (i.e., , where $n$ is the c index of refraction of the lasing medium and c is the speed of light) is substantially less than the pulse duration of the input pulse (i.e., less than the pulse duration of the pulses generated by the pump radiation source 71). For example, in some aspects, the roundtrip time can be less than 5 times shorter than the duration of the picosecond pump pulses input into the resonant cavity (e.g., less than 10 times shorter). Without being bound by any particular theory, it is believed that by shortening the resonant cavity, the output pulse extracted from the resonant cavity can have an ultra-short duration without the need for additional pulse-shaping (e.g., without use of a modelocker, Q-switch, pulse picker or any similar device of active or passive type). For example, the pulses generated by the wavelength-shifting resonator can have a pulse duration less than 1000 picoseconds (e.g., about 500 picoseconds, about 750 picoseconds).

After the picosecond laser pulses are extracted from the wavelength-shifting resonator 72, they can be transmitted directly to the treatment beam delivery system 73 for application to the patient's skin, for example, or they can be further processed through one or more optional optical elements shown in phantom, such as an amplifier 74, frequency doubling waveguide 75, and/or filter (not shown) prior to being transmitted to the treatment beam delivery system. As will be appreciated by a person skilled in the art, any number of known downstream optical (e.g., lenses) electro-optical and/or acousto-optic elements modified in accordance with the present teachings can be used to focus, shape, and/or alter (e.g., amplify) the pulsed beam for ultimate delivery to the patient's skin to ensure a sufficient laser output, while nonetheless maintaining the ultrashort pulse duration generated in the wavelength-shifting resonator 72. For example an optical element 76 (in phantom) can include one or more foci in, for example, the form of a lens array such as a diffractive lens array.

Lasers are recognized as controllable sources of radiation that are relatively monochromatic and coherent (i.e., have little divergence). Laser energy is applied in an ever-increasing number of areas in diverse fields such as telecommunications, data storage and retrieval, entertainment, research, and many others. In the area of medicine, lasers have proven useful in surgical and cosmetic procedures where a precise beam of high energy radiation causes localized heating and ultimately the destruction of unwanted tissues. Such tissues include, for example, subretinal scar tissue that forms in age-related macular degeneration (AMD) or the constituents of ectatic blood vessels that constitute vascular lesions.

Most of today's aesthetic lasers rely on heat to target tissue and desired results must be balanced against the effects of sustained, elevated temperatures. The principle of selective photothermolysis underlies many conventional medical laser therapies to treat diverse dermatological problems such as unwanted hair, leg veins, port wine stain birthmarks, and other ectatic vascular and pigmented lesions. The tissue layers including the dermal and epidermal layers containing the targeted structures are exposed to laser energy having a wavelength that is preferentially or selectively absorbed in these structures. This leads to localized heating to a temperature that denatures constituent proteins and/or disperses pigment particles (e.g., to about 70 degrees C). The fluence, or energy per unit area, used to accomplish this denaturation or dispersion is generally based on the amount required to achieve the desired targeted tissue temperature, before a significant portion of the absorbed laser energy is lost to diffusion. The fluence must, however, be limited to avoid denaturing tissues surrounding the targeted area.

Fluence is not the only consideration governing the suitability of laser energy for particular applications. The pulse duration (also referred to as the pulse width) and pulse intensity, for example, can impact the degree to which laser energy diffuses into surrounding tissues during the pulse and/or causes undesired, localized vaporization. In terms of the pulse duration of the laser energy used, conventional approaches have focused on maintaining this value below the thermal relaxation time of the targeted structures, in order to achieve optimum heating. For the small vessels contained in portwine stain birthmarks, for example, thermal relaxation times and hence the corresponding pulse durations of the treating radiation are often on the order of hundreds of microseconds to several milliseconds.

Cynosure's PicoSureTM brand laser system, which entered the commercial market in late March 2013 is the first aesthetic laser system to utilize picosecond technology that delivers laser energy at speeds measured in trillionth of seconds ($10^{-12}$). An exemplary PicoSure™ brand picosecond laser apparatus is detailed in our U.S. Patent Nos. 7,586,957 and 7,929,5 79. A picosecond laser apparatus provides for extremely short pulse durations, resulting in a different approach to treating various conditions than traditional photothermal-based treatments. Picosecond laser pulses have durations below the acoustic transit time of a sound wave through targeted tissues and are capable of generating both photothermal and photomechanical (e.g., shock wave and/or pressure wave) effects through pressures built up in the target.

Clinical results on tattoo removal with these systems show a higher percentage of ink particle clearance, which is achieved in fewer treatments. PicoSure™ picosecond laser systems can deliver both heat and mechanical stress (e.g., shock waves and/or pressure waves) to shatter the target ink particles from within before any substantial thermal energy can disperse to surrounding tissue. PicoSure picosecond laser systems, employing Pressure Wave™ technology, are useful for other applications including other aesthetic indications such as dermal rejuvenation, as well as other therapeutic applications where an increase in vascularization is desirable.

Blast injuries caused by detonation of explosives are known to cause shock waves and/or pressure waves that cause primary injuries that can damage a person's body including the lung, brain, and/or gut. Primary blast injuries are caused by blast shock waves and/or pressure waves. These are especially likely when a person is close to an exploding munition, such as a land mine. The ears are most often affected by the overpressure, followed by the lungs and the hollow organs of the gastrointestinal tract. Gastrointestinal injuries may present after a delay of hours or even days. Injury from blast overpressure is a pressure and time dependent function. By increasing the pressure or its duration, the severity of injury will also increase.

In general, primary blast injuries are characterized by the absence of external injuries; thus internal injuries are frequently unrecognized and their severity underestimated. According to the latest experimental results, the extent and types of primary blast-induced injuries depend not only on the peak of the overpressure, but also other parameters such as number of overpressure peaks, time-lag between overpressure peaks, characteristics of the shear fronts between overpressure peaks, frequency resonance, and electromagnetic pulse, among others. There is general agreement that implosion, inertia, and pressure differentials are the main mechanisms involved in the pathogenesis of primary blast injuries.

Thus, the majority of prior research focused on the mechanisms of blast injuries within gas-containing organs/organ systems such as the lungs, while primary blast-induced traumatic brain injury has remained underestimated. Blast lung refers to severe pulmonary contusion, bleeding or swelling with damage to alveoli and blood vessels, or a combination of these. Blast lung is the most common cause of death among people who initially survive an explosion. Applicants have surprisingly discovered that the shock waves and pressure waves that are known to harm organs and organ systems in a primary blast injury can be scaled down and controlled to provide systems and methods for controlled damage of cells and tissues (e.g., organs) that leads to improvement in the cells and tissues, improvements including tissue rejuvenation.

Laser Induced Optical Breakdown

Very short and high peak power a very short pulse width range from about 150 picoseconds to about 900 picoseconds, from about 200 picoseconds to about 500 picoseconds, or from about 260 to about 300 picoseconds comprised of deeply penetrating wavelengths (e.g., wavelengths such as that obtained with a 755nm alexandrite laser and/or a 1064nm NdYAG laser) may be focused at a depth in target tissues with the purpose of causing a laser induced optical breakdown (LIOB) injury. This LIOB injury features plasma initiated rapidly expanding bubbles in some pressure regimes these rapidly expanding bubbles are cavitation bubbles. At least a portion of the tissue within rapidly expanding bubble (e.g., the cavitation bubble) is near-instantaneously vaporized providing an ablation volume. Adjacent the vaporized volume are a roughly spherical injury where the most intense pressure waves called shock waves are concentrated.

Shock waves are the first portion of a high pressure expansion that extend away from the surface of the cavitation bubble through proximal tissues and cells. The shock waves that initially emanate from the cavitation bubble attenuate as they propagate through proximal tissues and cells experiencing a reduction in pressure and velocity and are then referred to as pressure waves. The shock waves are pressure waves that travel faster than the speed of sound and are believed to exhibit non-linear behavior. Shock waves attenuate into pressure waves when they travel at the speed of sound or less than the speed of sound. The behavior creates regions of shock waves (and resulting relatively intense mechanical stress on tissue and/or intense cell damage) nearer the cavitation bubble and regions of relatively reduced intensity pressure waves (and relatively reduced mechanical stress on tissue and/or reduced cell damage) as the distance from the cavitation bubble increases.

Figure 1B depicts a tissue injury 100 caused by the picosecond laser. At least a portion of the cavitation bubble 101 is ablated (e.g., vaporized) and in this pressure bubble 101 the photo thermal effect (e.g., temperature rise) of the picosecond laser on the tissue is largely confined. Biologic tissues and cells proximal to the surface of the cavitation bubble (ablation volume) therefore are exposed to the most intense shock wave region. Regions of tissues and cells farther from the cavitation bubble injury therefore are subject to ever decreasing pressure waves (e.g., ever decreasing magnitude pressure waves). This results in layers of cell damage not unlike layers of an onion, wherein layers of cells and tissue 102 more proximal to the cavitation bubble 101 experience the most intense pressure in shock waves and layers of cells and tissue more external to the bubble are subject to less intense pressure in pressure waves (e.g., cell layer 104 is exposed to less intense pressure than cell layer 103).

Referring still to Figure 1, the injury 100 is comprised of a central cavitation bubble 101 at least a portion of which has an ablation volume surrounded by tissue regions of relatively high cellular damage 102 having the most damage outside the cavitation bubble 101 with tissue layer 102 having the most cell damage, for example, total damage and immediate cell death, which are in turn surrounded by tissue layers 103, 104 and 105 having progressively lower cellular damage such that longer term cell death occurs with each progressively outer layer. For example, tissue layer 103 having severe cell damage (e.g., from about 1 to about 2 days until cell death), tissue layer 104 having moderate cell damage (e.g., from about 2 to about 7 days until cell death), and tissue layer105 having minor cell damage (e.g., from about 7 to about 21 days until cell death).

For example, layer 104 has a longer term cell damage (e.g., where cell death takes from about 2 to about 7 days) than layer 103 (e.g., where cell death takes from about 1 to about 2 days). The exemplary cell death dates are illustrative. Without being bound to any single theory, Applicants believe that it is important in that *ongoing deaths* of damaged cells which extend at least for several days and possibly for several weeks after the injury, are believed to enhance healing by continuing to deposit dead cell matter including proteins into nearby tissues. This ongoing long term cell death results in a longer duration of new cell genesis stimulated by the ongoing presence of cellular debris.

As the period of cell deaths extends, the period of presence of precursors for new cells is extended leading to a longer duration of stimulated new cell formation near the injury site, thereby improving healing and outcomes. It is believed that a sustained inflammatory period with ongoing release of cellular debris including cell proteins yields a longer period of new cell stimulation, a longer period of repair, and better healing compared, for example, to known photo thermal treatments (e.g., thermal laser treatments such as fractional photothermolysis).

The non-thermal effect (e.g., pressure wave and/or shock wave effect) of the cavitation bubbles are distinct from the pure photothermolysis effect resulting from laser irradiation. Photothermolysis does govern the underlying absorption of the applied laser pulse that forms the cavitation bubbles. Nevertheless, the non-thermal effects (e.g., shock waves and/or pressure wave and/or mechanical effects) are believed to create onion-like layers of lesions having varying amounts of cell damage within the target tissues.

The use of other short pulse lasers is common in ophthalmology lasers today. Ophthalmology applications rely on multi-photon ionization to create LIOB's in transparent media common to ophthalmology. In general, ophthalmology applications prefer use of femtosecond pulse widths, which provide a relatively precise ablation bubble injury that that reduces unwanted shockwave injury of tissue adjacent to the ablation volume. Femtosecond initiated LIOB's limit and/or avoid photomechanical (e.g., shockwave and pressure wave) and photothermal effects outside of the ablation bubble. The femtosecond initiated LIOB's are effective at using the LIOB energy to thoroughly ablate the internal bubble volume leaving little to no energy to escape outside the bubble as photomechanical energy (e.g., shockwaves and/or pressure waves). Such precision is paramount in ophthalmology applications to ensure integrity of the eyes.

Conversely, in our application employing the pulse widths that range from about 190 picoseconds to about 900 picoseconds, from about 200 picoseconds to about 500 picoseconds, or from about 260 to about 300 picoseconds, the cavitation bubble injury is mediated by shockwaves and by pressure waves, which create the desired injury. With pulse widths from about 190 picoseconds to about 900 picoseconds, from about 200 picoseconds to about 500 picoseconds, or from about 260 to about 300 picoseconds, the cavitation bubble expansion and the resulting mechanical damage (e.g., shock waves and/or pressure waves) all contribute to the mechanism of action. More specifically, pulse widths from about 190 picoseconds to about 900 picoseconds, from about 200 picoseconds to about 500 picoseconds, or from about 260 to about 300 picoseconds can be employed to induce micro injuries that are mediated by plasma explosion initiated cavitation bubbles and the resulting shock waves and pressure waves.

Once initiated, the laser induced plasma absorbs the laser radiation and thus couples the incident energy efficiently into the material. In other words, once the plasma forms the rest of the laser pulse energy is efficiently coupled into either thermal effect in the case of nanosecond pulse widths or in the case of picosecond pulse widths into mechanical forces caused by shockwaves and pressure waves. In a picosecond application the mechanical forces cause the bulk of the injury as opposed to a temperature rise.

Habbema et al (J. Biophotonics, 5, No. 2, 194-199: 2012) disclose an LIOB device and method intended to treat tissue by means of plasma mediated ablation to stimulate new collagen growth. However, Habbema concentrates on the ablated region and neglects the critical role of the pressure wave treated volumes and the tissue layers of varying length of cell death. Habbema focuses as the ablated or vaporized volume. The Habbema paper prefers shorter pulsewidths into the femtosecond domain as they emphasize a very confined and controlled region of LIOB injuries (specifically, the vaporized volume). Habbema references ophthalmic applications of femtosecond pulses in transparent tissues, applications that primarily intend to ablate tissue in precise fashion and seek to deliberately minimize effect's to adjacent tissue located outside the vaporized volume.

In contrast, our application having pulse widths from about 190 picoseconds to about 900 picoseconds, from about 200 picoseconds to about 500 picoseconds, or from about 260 to about 300 picoseconds essentially sacrifices a small region of the tissue that becomes the cavitation bubble containing the vaporized volume as a means to generate mechanical forces including shock waves and pressure waves to disrupt a much larger external volume than the LIOB (vaporized volume) injury alone. The femtosecond pulses initiate a more intense multi-photon avalanche mechanism which so efficiently ablates tissue that little to no energy escapes to surrounding tissues as pressure waves. In this way, the femtosecond pulse width energy is "neatly" contained where in contrast the pulse width from about 190 picoseconds to about 900 picoseconds, from about 200 picoseconds to about 500 picoseconds, or from about 260 to about 300 picoseconds provides energy in a "sloppy" manner that applies shock wave and pressure wave energy of varying intensities in tissue layers outside the cavitation bubble.

Oraevsky et al (IEEE Journal of Selected Topics in Quantum Electronics, Vol. 2, No. 4, December 1996, 801-809) describes picosecond pulsewidth LIOB's in high absorbance tissue with a focus toward the "as short as possible" femtosecond pulse widths to confer the most predictable lesion size and reduced collateral damage. Oraevsky discloses data which indicate the reduced utility of femtosecond pulses as compared to picosecond pulses for pressure wave generation, although Oraevsky fails to recognize that picosecond pulses are capable of generating maximized pressure waves. Instead Oraevsky focuses on the higher ablation efficiencies achievable with femtosecond pulses due to multi-photon ionization, thereby ignoring the contribution of the pressure wave generation by picosecond pulse widths.

Applicants believe that it is possible to preferentially make use of electron ionization and its resulting avalanche as the main process which drives the explosive plasma expansion in picosecond driven LIOB's as well as the consequent mechanical waves (e.g., shock waves and/or laser pressure waves) creating regions of lessening mechanical damage as the distance from the cavitation bubble is increased.

The picosecond LIOB differs from nanosecond LIOB in several ways. Due to the nanosecond pulse being relatively longer ($10^{-9}$) than the picosecond pulse ($10^{-12}$). It takes longer to accumulate energy in an absorptive center of a cavitation bubble with nanosecond as compared to the time it takes using picosecond laser pulse. Therefore nanosecond pulses precipitate lower magnitude pressure waves having a less steep rising edge, which reduces the peak pressure wave stresses imparted to adjacent tissues (e.g., adjacent tissue layers) as compared to the relatively intense peak pressure waves imparted on adjacent tissue layers by a picosecond laser pulse. Thus, picosecond pulses are more efficient at coupling steep rising pressure waves into tissue compared to nanosecond pulses. Oraevsky et al teaches that the ionization threshold fluence is relatively independent of pulsewidth for strongly absorbing gel's (target chromophores in our example). Oraevsky says "The laser threshold fluence is largely independent of pulse duration for strongly absorbing gels". (Oraevsky Section IV. Experimental results). Thus for very high absorption areas a correspondingly longer pulse duration (longer meaning picosecond or short nanosecond opposed to femtosecond) will suffice to initiate LIOB's. Picosecond pulses however, efficiently convert the ablation expansion or LIOB energy into therapeutic shockwaves that attenuate into pressure waves. In contrast nanosecond or femtosecond pulse durations provide energy that creates the cavitation bubble. Picosecond pulse durations therefore confer the ability to treat larger volumes of tissues, cells or targets with LIOB injuries, than is possible with femtosecond pulses of equivalent or even greater fluence. This is true because the volume of tissue treated or injured by picosecond duration LIOB's is greater than the volume treated by femtosecond duration LIOB's due to the greater magnitude and greater effective radius of shockwaves and pressure waves possible with picosecond LIOB's. Femtosecond ablation has such a high ablation efficiency and such a steep wavefront that shockwave and pressure wave effects are retarded at these pulse durations. Femtosecond duration LIOB's are ideal for ophthalmologic or other applications where only ablation is desired and where minimal adjacent tissue damage is desired.

It should be noted that a greater range of cell types may also be treated with picosecond LIOB initiated shockwave and pressure wave injuries including tissues, cells or targets with absorption too low to allow picosecond duration LIOB formation for a given fluence. This is possible provided these greater range of tissues, cells and targets are within the effective shockwave and pressure wave radius of a strongly absorbing target/chromophore which allows picosecond LIOB formation at a given fluence.

As discussed previously, shock waves and pressure waves of varying intensity propagate through the tissue as a result of a picosecond LIOB injury being imparted on the tissue. The propagation of these intense waves through biologic tissues manifests as mechanical stress and strain in the tissue cells. Susceptibility to mechanical stress and/or cellular damage varies depending on the cellular structure. The susceptibility of tissues containing gas or air volumes to pressure waves and/or shock waves is especially pronounced. For example, pulmonary tissues are examples of tissues containing gas or air volumes that may be treated in accordance with the methods and devices disclosed herein. It is believed that picosecond lasers may provide a tool for lung disease treatment including chronic obstructive pulmonary disease (COPD) treatment/therapy and are promising for regeneration of myocardium surface tissues where improved vascularization and/or reduction in the stiffness of scar tissues is desirable.

Fibrous tissues are also excellent targets for picosecond LIOB pressure wave and/or shock wave mediated therapies. For example, it is believed that more elastic tissues and more elastic cells exposed to picosecond pulses are likely to have a higher peak pressure damage threshold as compared to more rigid or fibrous cells or tissues. Correspondingly, LIOB pressure wave initiated damage will tend to accumulate tissues that are more rigid, often fibrous, and less flexible. In particular, collagen fibers and other fibrous tissues are believed to be more likely to experience pressure wave initiated damage. Fibrous cells such as collagen, elastin, and bone tissue are believed to be susceptible to pressure wave initiated damage to an extent greater than more elastic cells and tissues.

Neural tissues are believed to be excellent targets for picosecond LIOB shockwave and pressure wave mediated therapies. Since the disclosed picosecond treatment strategy depends on the creation of precise micro-lesions, tissues which are more susceptible to shock wave injuries should therefore respond more readily, easily and efficaciously to picosecond LIOB's. For example, nerve cells are likely more susceptible to shockwave and pressure wave mediated therapy.

In some embodiments, the practitioner targets treatment parameters with the picosecond laser based, at least in part, on the susceptibility of tissue areas and/or cell types to photo thermal damage and/or to pressure damage. Thus, the treatment can be guided by an understanding of cell and tissue susceptibility to shockwaves and/or pressure waves and to temperature rise. Other tissues that may be treated with the picosecond laser include, for example, lungs, bowel, colon, throat, dermis, or any other tissue accessible by the laser output.

The picosecond laser is especially useful to treat tissue affected by scarring and/or loss of flexibility due to previous injury or infection. The picosecond laser is especially useful to alter and/or to reduce the stiffness of tissues including scar tissues. Scar tissue and other stiffened tissues inhibit the ease of movement necessary to enable body function, and/or organ function and/or for comfortable limb function.

A shockwave and/or pressure wave injury provides an entirely different mechanism of action as compared to thermal injury wherein the shockwave and/or pressure wave disrupts, tears and breaks cells and cellular contents. Shearing forces generated by, for example, mechanical forces (e.g., shockwaves and/or pressure waves) can break collagen and other fibers as well as rupture other cell types. The resulting cellular debris from ruptured cells as well as signals from substantially injured cells triggers a regeneration of tissue more representative of normal uninjured tissue. One example of a regeneration result would be the treatment of dermal scar tissue with about 300 picosecond LIOB initiated shockwaves and pressure waves delivered by means of a micro-lens array. Scar tissue may be broadly characterized as having a different ratio of collagen types as well as having fewer and or smaller elastin cells, as compared to normal un-scarred tissue. In this case, LIOB shockwave pressure injured tissue stimulates the regeneration of a more normal balance or ratio of elastin and collagen types. This results in tissue that is softer smoother and better feeling (to the patient) and to a reduced scar.

Another example of regenerated tissue expected results would be applications involving pulmonary or myocardium tissue, both subject to numerous degenerative diseases which involve hardening tissues, scarring, and/or a reduction in flexibility and mobility. By improving the mechanical flexibility of degenerated pulmonary and/or myocardium tissues, an improvement in organ function and thus patient well-being is possible. In this example picosecond LIOB micro-injuries initiate tissue regeneration similarly to the dermal scarring example above wherein the resulting regenerated tissue will consist of a more normal balance of fibrous and elastic cell types including ratios of collagen types and number and size of elastin and other elastic cells. The result is softer, more flexible, more functional regenerated organs. There are numerous other organs and tissue types susceptible to reduced function due to scarring, all of which may benefit from picosecond mediated mechanical injuries (e.g., shockwave and/or pressure wave). Such tissues and organs may be treated by a handpiece including a single beam (e.g., a single fiber), or alternatively by a beam that traverses a microlens array or a scanner that provides two or more picosecond pulses separated by untreated tissue (i.e., treats the tissue fractionally).

In one embodiment, in micro-fracture orthopedic procedure bone ends are fractionally drilled to promote improved vascularization to feed cartilage. The procedure thickens and strengthens cartilage by using deeper and more vascularized bone portions to feed cartilage in the bone. Picosecond initiated LIOB in a micro-fracture orthopedic procedure benefits this process by simultaneously ablating a channel (e.g., a micro-drill like hole) while injuring proximal to bone cells with shockwaves and pressure waves. It is believed that stacked pulses delivered in the picopulse regime by a scanner could facilitate LIOB ablation drilling of cylindrical holes, which leads to enhanced healing due to a longer term "pressure onion" injury as well as general LIOB benefit's including a propensity to stimulate angiogenesis. In this way, the micro-fracture orthopedic procedure can initiate improved vascularization for cartilage regrowth.

Control of applied pulse energy and to a lesser extent pulse duration provides control of lesion size. Use of an appropriate lens array can provide control of lesion depth. This allows the clinician to create precise injuries in precise locations in the target tissue. By use of a lens array, the fluence may be further intensified and/or focused beneath the tissue surface, for example using a fractional array as described in U.S. Patent No. 6,997,923 which provide focused regions of tissue separated by untreated or less treated tissue or using a CAP array as described in U.S. Patents 7,856,985, 8,322,348 and 8,317,779 (incorporated by reference herein) which provides for a non-uniform output beam having high and low fluence zones. Likewise, different lens arrays will focus the laser and therefore permit creation of tissue lesions at precise depths. For example, a $100\mu$m depth array may be complemented by $450\mu$m, $750\mu$m, $1000\mu$m, or $2000\mu$m depth arrays, thereby allowing for treatment of tissues having thicker cross sections. Interlaced differing focus depth lens arrays also allow for novel and very precise injuries within tissue. A single array could embody or allow a multiplicity of different focus depth lenses in whatever pattern is desired, perhaps allowing for subsurface curving or tightening of tissues by providing a bias to the injury patterns. Interlaced cross stitch patterns of varying depth are also possible.

Dermal Rejuvenation

For skin rejuvenation procedures Habbema discloses formation of 0.1 to 0.2mm lesions formed between 100-750$\mu$m beneath the epidermis surface when using a 150$\mu$J sub-nanosecond laser at a wavelength of 1064nm when focused into a 10$\mu$m focal spot. Habbema shows histology indicating the presence of dense clusters of erythrocytes proximal to lesion sites within skin tissue 30 minutes after irradiation. In addition, 30 days post treatment Habbema shows histologcal evidence for new collagen formation. However, the device of Habbema is limited in that the relatively lower energy output by the device limits its use to small treatment areas. An exemplary system for dermal rejuvenation is described by our PicoSure™ brand picosecond laser apparatus detailed in our U.S. Patent Nos. 7,586,957 and 7,929,579, which provides a 200mJ/pulse as compared to the 0.15mJ/pulse used by Habbema, and generates a more than 1333-fold energy increase, which allows for treating larger areas and faster treatment times. The PicoSure™ apparatus generates pulsed laser energy having a pulse duration of about 220-900 picoseconds. Laser energy having a wavelength in the range of 500-1100 nm provides excellent specificity for collagen, and the major chromophores of skin, permitting the rapid formation of plasma, resulting in dermal lesions due to cavitation. Treatment times will vary according to the desired effects.

Taking a picosecond laser emitting a pulse width that ranges from about 260 picoseconds to about 900 picoseconds, from about 300 picoseconds to about 775 picoseconds, from about 450 picoseconds to about 600 picoseconds, or from about 260 picoseconds to about 300 picoseconds and modifying the output beam via fractional technology as described in U.S. Patent No. 6,997,923 or modifying the output beam via CAPS technology as described in U.S. Patent No. 7,856,985 provides a particularly useful approach to rejuvenating tissue and inducing collagen and epithelial cell restoration within the tissue. In an embodiment where a non-uniform output beam is delivered to tissue from a source of light as described in our patent applications U.S.S.N. 11/347,672; 12/635,295; 12/947,310, and PCT/US10/026432.

The non-uniform beam is characterized by a cross-section corresponding to an array of relatively small, relatively high-fluence, spaced-apart regions superimposed on a relatively large, and relatively lower-fluence background. Operatively, this produces within the area of the beam, regions of relatively greater energy and relatively lower energy. Exemplary temperature dependent effects include but are not limited to parakeratosis, perivascular mononuclear infiltration, keratinocyte necrosis, collagen denaturation, and procollagen expression in dermal cells. Other cellular markers (e.g., nucleic acids and proteins) are useful in detecting more subtle responses of skin to less aggressive treatments. Exemplary photomechanical effects include the formation of lesions within the dermis. Erythrocytes accumulate in the damaged areas, and a healing response ensues, with consequent collagen formation and rejuvenation of the dermal tissue.

The overall effect of treatments on skin tone, wrinkling and pigmentation provide the best indication of therapeutic efficacy, but such treatments also leave histological evidence that can be discerned. At the highest energies, lesions due to plasma formation and cavitation are detectable. At higher energies (and especially at longer pulse durations in the nanosecond range), the thermal damage is easy to detect. For more moderate energies, microthermal damage can produce effects that are seen with magnification although erythema provides a good marker for microthermal injury and it does not require microscopic examination of tissues from the treatment site. Generally, in the absence of any visually observable erythema, the cellular effects will be more subtle, or may take longer to manifest themselves or may require multiple treatments before visual improvement of the skin is seen. At lower output energies, shorter pulse durations, and longer intervals between treatments, it is advantageous to use more sensitive techniques to assay for cellular changes.

Certain techniques provide for quantitative analysis, which are correlated to describe a dose-response relationship for the non-uniform beam, as it is used in dermal rejuvenation applications. Such techniques include but are not limited to RT-PCR and/or real-time PCR, either of which permits quantitative measurements of gene transcription, useful to determine how expression of a particular marker gene in the treated tissues changes over time. In addition to nucleic acid-based techniques, quantitative proteomics can determine the relative protein abundance between samples. Such techniques include 2-D electrophoresis, and mass spectroscopy (MS) such as MALDI-MS/MS and ESI-MS/MS. Current MS methods include but are not limited to: isotope-coded affinity tags (ICAT); isobaric labeling; tandem mass tags (TMT); isobaric tags for relative and absolute quantitation (iTRAQ); and metal-coded tags (MeCATs). MeCAT can be used in combination with element mass spectrometry ICP-MS allowing first-time absolute quantification of the metal bound by MeCAT reagent to a protein or biomolecule, enabling detection of the absolute amount of protein down to attomolar range. Modifying the picosecond output beam utilizing a fractional approach by which focused regions of treated tissue are separated by untreated or less treated tissue can yield dermal rejuvenation effects similar to those described in view of the non-uniform beam CAPS array approach.

Scar Tissue/Striae

Scarring, striae and other harder to treat tissues also benefit from such photomechanical treatments. Exemplary non-limiting types of tissues include hypertrophic scars, keloids and atrophic scars. Hypertrophic scars are cutaneous deposits of excessive amounts of collagen. These give rise to a raised scar, and are commonly seen at prior injury sites particularly where the trauma involves deep layers of the dermis, i.e., cuts and burns, body piercings, or from pimples. Hypertrophic scars commonly contain nerve endings are vascularized, and usually do not extend far beyond the boundary of the original injury site. Similarly, a keloid is a type of scar resulting from injury, that is composed mainly of either type III or type I collagen. Keloids result from an overgrowth of collagen at the site of an injury (type III), which is eventually replaced with type 1 collagen, resulting in raised, puffy appearing firm, rubbery lesions or shiny, fibrous nodules, which can affect movement of the skin. Coloration can vary from pink to darker brown. Atrophic scarring generally refers to depressions in the tissue, such as those seen resulting from Acne vulgaris infection. These "ice pick" scars can also be caused by atrophia maculosa varioliformis cutis (AMVC), which is a rare condition involving spontaneous depressed scarring, on the cheeks, temple area and forehead.

Existing laser treatments are suitable for hypertrophic and atrophic scars, and keloids, and common approaches have employed pulsed dye lasers in such treatments. In raised scars, this type of therapy appears to decrease scar tissue volume through suppression of fibroblast proliferation and collagen expression, as well as induction of apoptotic mechanisms. Combination treatment with corticosteroids and cytotoxic agents such as fluorouracil can also improve outcome. In atrophic scars, treatments can even out tissue depths.

Striae (stretch marks) are a form of scarring caused by tearing of the dermis. They result from excess levels of glucocorticoid hormones, which prevent dermal fibroblasts from expressing collagen and elastin. This leads to dermal and epidermal tearing. Generally, 585-nm pulsed dye laser treatments show subjective improvement, but can increase pigmentation in darker skinned individuals with repeated treatments. Fractional laser resurfacing using scattered pulses of light has been attempted. This targets small regions of the scar at one time, requiring several treatments. The mechanism is believed to be the creation of microscopic trauma to the scar, which results in new collagen formation and epithelial regeneration. Similar results can be achieved, albeit to the total scar, through the use of modified laser beams as described in our U.S. Patent No. 7,856,985, detailing the use of non-uniform beam radiation to create within the beam area, discrete microtrauma sites against a background of tissue inducing laser radiation.

An exemplary system for treating scars is described by the above apparatus generating pulsed laser energy having a pulse duration of about 100-500 ps with about 100-750 mJ/pulse. Laser energy having a wavelength in the range of 500-1100 nm provides excellent specificity for collagen. Photomechanical disruption of the scar tissue is effected using the short pulse duration (below the transit time of a sound wave through the targeted tissue), together with a fluence in the range of 2-4 J/cm$^2$. This fluence is achieved with a laser energy spot diameter of about 5 mm, which can be changed according to the area of the target. Treatment times will vary with the degree of scarring and the shapes of the targets. Modifying the output beam as described in U.S. Patent No. 7,856,985 provides a particularly useful approach to reducing scar appearance and inducing epithelial restoration within the scar.

In a sub-surface picosecond induced LIOB injury, vaporized material remains in the vaporized cavitation bubble as it is a closed below the epidermis. One difference between the picosecond approach and other available laser scar therapies is that after the ablative or denaturing injury, the treated tissue typically remains open to the environment. In contrast, a sub-surface picosecond induced LIOB lesion leave vaporization products (e.g., the ablated cellular debris) in the ablated cavity. Applicants believe that these cellular debris remaining in the injury cavity trigger enhanced phagcytotic activity of macrophages. The presence of abundant macrophages in tissues healing after LIOB injuries has been noted in the literature (Habbema et al). In this way, cellular debris trapped in the LIOB cavity "enhance healing" as compared to ablated and removed material, as is common with the prior purely or substantially photothermal laser approaches to scar modification that require longer pulse widths.

Another important difference between picosecond LIOB and other laser therapies for scar modification is that the energy coupled to the target tissue via the cavitation bubble is mediated by shock waves and pressure waves that travel outside the cavitation bubble. The mechanical damage is in contrast to photothermal temperature rise effects typically employed in prior laser treatment approaches. This is important as thermal treatments, i.e., longer pulsewidth therapies mediate energy to the target tissue by thermal means and this can result in unwanted/undesirable additional thermal damage to adjacent regions. As shock waves and pressure waves propagate away from the LIOB site the pressure waves reduce in intensity. In this way, in the tissue regions more distant from the LIOB, cell types that are sensitive to the impact of pressure waves will sustain damage while less sensitive cell types (e.g., less susceptible cell types) remain less damaged and/or essentially undamaged. Tissue types and cell types that are sensitive to the impact of pressure waves can, in this way, be selective to the pressure waves caused by picosecond LIOB.

In some embodiments, picosecond LIOB is utilized so that shock waves and pressure waves are preferentially developed to form targeted injuries. Preferentially developing pressure waves is in contrast to maximizing ablation efficiency (e.g., maximizing the ablated volume as may be done when using femtosecond pulse driven LIOB's). The reduced "ablation efficiency" of picosecond laser pulses as compared to femtosecond pulses may create an injury superior for tissue rejuvenation. In other words, the greater magnitude of shock waves and pressure waves achievable with picosecond pulses as compared to shorter femtosecond pulse are more suited to cause a more desirable injury. In the case of longer nanosecond pulses, insufficient electron ionization avalanche intensity as compared to picosecond pulses results in less intense pressure waves. In this way picosecond pulses are more suited for creating mechanical wave (e.g., shock wave and/or pressure wave) injuries including a central cavity (ablation volume) surrounded by tissue regions of high cellular damage (short term cell death), which are in turn surrounded by low cellular damage regions wherein longer term cell death occurs and with regions of healthy undamaged tissue beyond the low damage region.

Without being bound to any single theory, it is believed that the graduated "onion style" regions of decreasing damage caused by high intensity shock waves that attenuate into lower intensity pressure waves can offer features of a wound which stimulates a sustained cellular repair period (e.g., up to weeks long). In other words, the pressure wave portion of the injury creates damaged, but not immediately killed cells (as depicted in Figure 1). The pressure wave mediated portions of the injury, as opposed to the ablated region, provide injury features uniquely well suited to tissue rejuvenation. Applicants suspect the picosecond induced pressure wave injury stimulates more collagen regrowth than fully ablated therapies such as femtosecond laser pulses with consequent multi-photon ionization. It is also possible to form complex 3 dimensional geometry patterns of "injuries", below the tissue surface, for the purpose of creating a bias in rejuvenated tissue such as support structures (built by new collagen), cross stiches, rows of injuries designed to cause contraction along an axis once it has healed.

Other Tissues

Lesions in the dermis created using higher energy systems permit more aggressive treatment, but LIOB mediated mechanical waves (e.g., shock waves and/or pressure waves) also find application with treating harder and less vascularized tissues (e.g., ligaments and cartilage) and improving their healing and regeneration. Stimulated or enhanced healing of all tissues, not just skin, is possible by the apparatus and methodologies described herein. LIOB mediated shock wave and pressure wave treatments recruit erythrocytes and stimulate tissue growth. Therefore, other potential re-vascularization applications that utilize LIOB to create initial tissue lesions that initiate a healing response include chronic wound care applications such diabetic related edema and other non-acute wound therapies. Typically such wounds can be characterized by poor vascularization which greatly complicates healing. Burn wounds are another potential therapeutic application. Additionally there are a number of liver and circulatory disorders including calcification of vasculature, which are candidates for re-vascularization therapies based on the creation of LIOB channels.

As an adjunct therapy performed after the primary surgery on an organ, LIOB channel injuries that mediate shock wave and pressure wave injuries may be applied to promote revascularization in areas of the organ where vascularity is of concern. Alternately non-channel style, or array based (spread out isolated injuries) may be applied to promote general healing.

Controlling pulse-width to select desired injury mechanisms

Short pulse wave (femtosecond, picosecond, & nanosecond) laser irradiation may be focused into high absorbance tissues or cells to initiate laser induced optical breakdown LIOB. Characteristics of LIOB induced injuries in dermal or epidermal tissue features, for example: a Vaporized volume (area within the expanded LIOB plasma bubble), a shock wave damaged area that attenuates into a pressure wave damaged area (surrounding the vaporized volume), and thermal energy (surrounding the vaporized volume). In the case of LIOB there will always be a vaporized volume, however, the generation of either shock waves and/or pressure waves and/or thermal energy/temperature rise will be strongly influenced by the selection of pulse width. In certain targeted injuries and/or tissue areas it may be advantageous to emphasize the thermal expression of energy to denature proteins, for example. Whereas in other targeted injuries and/or tissue areas it may be advantageous to emphasize the shock wave and pressure wave expression of the LIOB for the purpose of maximizing mechanical or pressure mediated injuries where, for example, it is desired to lessen and/or avoid denaturing proteins. In yet another application it is possible to obtain an advantage by emphasizing the expression of the vaporizing volume, thereby avoiding the expression of either thermal or pressure wave mediation of injuries. This manifestation of such a purely vaporized volume injury would be most advantageous for an ablative application wherein areas nearby and/or adjacent the plasma vaporized volume (ablated volume or cavitation volume) remain uninjured by either thermal deposition or shockwaves or pressure waves.

No cells will tolerate ablation however the tolerance that differing cell types have for either shock waves and/or pressure waves or thermal injury vary based on the cell type. Thus, some cell types and/or tissue types are more susceptible to damage by heating, and other cell types and/or tissue types are more susceptible to shock wave and/or pressure wave injuries. Further, the types of cells and/or cell constituents that are predominantly damaged can be preferentially selected by tuning the plasma expansion bubble rate of expansion. This allows the creation of very precise localized injuries mediated by either shock wave and/or pressure wave/mechanical forces or by thermal forces/ temperature rise depending on the clinical preference. Additionally, it is possible to avoid causing damage to very susceptible cell types, or to preferentially cause damage to susceptible cell types by controlling the pulse width to tune the expansion bubble such that it provides the desired shock wave and/or pressure wave magnitude (more or less), the desired thermal rise (more or less) or the desired combination of shock wave and/or pressure wave and thermal rise.

Controlling and/or tuning the plasma expansion bubble (e.g., the rate of expansion of the plasma expansion bubble) is accomplished by selection of pulse width used to initiate and drive the plasma bubble expansion. Controlling the ablated volume or lesion size is accomplished by selection of a fluence greater (more or less) than the ablation threshold. More specifically, the laser pulse energy in excess of the ablation threshold serves to expand the ablation bubble size. After avalanche breakdown has occurred any remaining laser pulse energy acts to expand the avalanche lesion size. Accordingly, a user's selection of higher relative fluence for a target will necessarily increase the laser energy delivered to the target area/lesion. Applicants believe that plasma expansion bubbles initiated by pulse widths in the femtosecond, picosecond, and nanosecond ranges act on tissue as follows:

1. Femtosecond duration pulses (and less than 220 picoseconds) initiate multi-photon ionization, which causes a very rapid expansion and a consequent evaporation of tissue. Femtosecond pulse waves and pulse waves less than 220 picoseconds provide a very clean and precise ablation bubble with minimum energy escaping beyond the ablation bubble. Thus, the magnitude of pressure waves escaping beyond an expansion bubble can be minimized by employing a relatively short pulse width, e.g., in the femtosecond range and less than 220 picoseconds. At less than about 220 picoseconds additional ionization mechanisms begin to act that simultaneously increase ablation efficiency while decreasing shockwave intensity. All the energy begins to get used up by creating the ablation cavity once multi-photon ionization processes begin at about 220 picoseconds.

2. Picosecond duration pulses (at about 260 to about 900 picoseconds, at about 260 to about 500 picoseconds, or at about 260 to about 300 picoseconds and above) do not initiate multi-photon ionization in tissue. Instead picosecond duration pulses (at about 260 to about 900 picoseconds, or at about 260 to about 300 picoseconds) rely on an electron ionization avalanche mechanism. The plasma expansion bubble forms (or cavitation bubble forms) and the regions surrounding the plasma expansion bubble are exposed to shock waves that attenuate and decrease in pressure into pressure waves, which further decrease at a distance from the cavitation bubble. The magnitude of the shock wave pressure reaches a maxima at around 260 picoseconds. Pulse durations above 260 picoseconds reduce in shockwave magnitude and pulse durations less than 260 picoseconds also reduce in shockwave magnitude, however, in the case of pulse durations less than about 220 picoseconds the reduction in shockwave magnitude is due to the onset of the more rapid multi-photon ionization in tissue.

3. Nanosecond duration pulses (and greater than about 900 picoseconds) also do not initiate multi-photon ionization, instead nanosecond duration pulses rely on an electron ionization avalanche mechanism. Nanosecond duration LIOB plasma expansion bubbles however, expand more slowly than picosecond duration LIOB bubbles and thus provide a less steep wavefront with a greatly reduced magnitude and a reduced radius of useful shock waves and pressure waves. Rather, energy not consumed in the LIOB ablation volume is principally delivered to the tissue as thermal energy.

The type of damage e.g., thermal mediated, shock wave mediated, pressure wave mediated, or combination thereof can be selected to influence the desired damage and/or to achieve a desired course of healing. This is important, because the composition or the makeup of replacement cells as the damaged tissues heal will impact and/or determine the outcome of the treatment. The way that the damaged cells heal is determined at least in part by the type of tissue injury whether mechanical (shockwave and/or pressure wave) or thermal and/or the combination of mechanical and/or thermal injury. By selectively damaging cells it is possible to select the ratio of cell types in newly healed tissues.

For example, burn injuries of dermal tissue typically result in the formation of scar tissue. Burn scar tissue is harder, tougher, and less flexible than unscarred dermal tissue. Also, scar tissue can be unsightly as well as a source of discomfort. Generally all scar tissue types have fewer and smaller elastin cells, additionally the ratio of collagen types expressed in "scar" tissue differs from healthy uninjured tissue. We presume that thermal damage to the cells in a burn injury provides characteristic cellular debris of a burn injury to the bloodstream and this triggers a typical or normal healing/regeneration result of scar tissue formation (de-emphasized elastin and different ratio of collagen types regenerated as compared to uninjured tissue). One could presume that the thermal damage of cellular contents and resulting cellular debris alters them to become less recognizable or less able to stimulate the full regeneration of a normal ratio of typical uninjured dermal tissue (including elastin).

A mechanical injury such as a shockwave injury and/or a pressure injury provides an entirely different mechanism of action for example the shockwave and/or the pressure wave disrupts, tears and breaks cells and cellular contents. Shearing forces generated by shockwaves and/or pressure waves can break collagen and other fibers. The resulting cellular debris from ruptured cells as well as signals from injured cells triggers a regeneration of tissue more representative of normal uninjured tissue, shockwave and pressure wave injured tissue stimulates the regeneration of a more normal balance or ratio of elastin and collagen types. Shockwave and pressure wave caused cellular debris remains unaltered by thermal effects, perhaps appearing to the bodies regenerative systems in a form (similar cellular debris constituents as in normal cellular attrition) which stimulates a more balanced, normal, regeneration as opposed to thermal injuries which are known to "scar".

Of particular note, a shockwave and/or pressure wave type of micro-injury will be far more likely to severely injure but not kill cells outright as compared to thermal injury. This extends the regenerative period for shockwave injuries which is believed to contribute to a better outcome (e.g., better regeneration).

Short pulse plasma expansion bubbles

Selective photothermolysis is a thermal approach to creating tissue injuries, by thermal means. In the picosecond and nanosecond domains, linear absorption and thus photothermolysis describe the initiation of ionization and consequent non-linear absorption, resulting in an expanding plasma bubble. It should be noted that shock wave-front maximum attainable pressures far exceed the magnitudes of commonly observed acoustic waves or popping sounds from short pulse laser linear photothermolysis. Shock waves and after some attenuation pressure waves from plasma bubble expansion do considerable tissue damage. Thus tissue treatment at very high energies and pulse widths in the picosecond and nanosecond domains that are short enough to initiate plasma bubbles and electron ionization avalanche providing a shock wave front requires a new paradigm.

These phenomena provide effects on living tissue. Pulse widths above about 260 picoseconds avoid initiating multi photon ionization. The maximum efficiency of shockwave generation will be between about 260-300 picoseconds. But operation on either side of that "peak" will still generate effective shockwaves. Oraevsky calculates that 350 femtsecond pulses generate 4 times lower recoil pressure amplitude and 2 times lower shock gradient than a comparable 350 picosecond duration pulse ("0.66-kbar for the 350-fs ablation and 2.6-kbar for the 350-ps ablation"). For our discussion: a better understanding is that as pulse durations ranging from about 50-100 nanseconds down to about 260 picoseconds provide continuum of effects, all of which may be useful. More precisely, at the low end of the pulse duration range e.g., about 260 picoseconds, maximum efficiency of shockwave energy generation is obtained. At the higher duration end of the scale in nanosecond regime, e.g., about 50-100 nanoseconds, maximum efficiency of thermal energy generation is obtained.

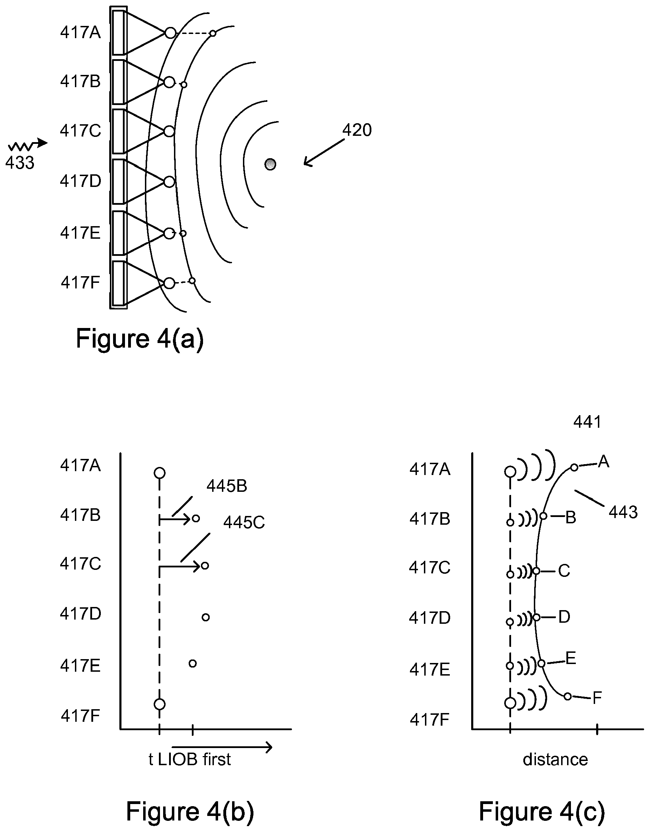

Reliable formation of plasma bubbles requires the irradiance to exceed the breakdown or ablation threshold. In a practical sense this requires spot size and/or Joules/pulse to be adjustable to match the selected pulse width. Peak energy densities of between 2-50 $J/cm^2$ will exceed the ablation threshold in collagen gels and porcine corneas (Oraevsky et al fig 4), and these are somewhat representative biological tissues.

Pulse width may be adjusted, tuned and or controlled to control the expansion rate of the ablation volume, which controls the expression of the bubble's expansion energy into either, predominantly shockwaves/pressure waves (260 picosecond to 300 picosecond) or predominantly thermal (>1 nanosecond) causing a temperature rise of tissue adjacent to the ablation volume. Selection of intermediate pulse widths between these two ranges apportions the energy between shockwave and thermal effects.

An example laser 755nm alexandrite has a pulse width from about 260 picoseconds to about 50 nanoseconds can be controlled or tuned depending on the desired outcome and/or injury combination. The exemplary alexandrite can employ pulse widths of about 260-500 picoseconds, which are selected to cause optimum shockwave injuries and pulse widths above about 1 nanosecond, which are selected to cause predominantly thermal injuries.

Sequential plasma expansion bubbles

Sequential overlapping pulses may be applied to tissue. At the end of the first plasma bubble expansion, and while this region of tissue remains ionized, a second pulse is fired and is readily absorbed by the first bubble's ionized region. This approach could yield relatively larger volume injuries. An application might include orthopedic micro fracture of bones to enhance vascularization. A typical longer pulse more thermal laser ablation approach may denature too much tissue and make re-vascularization to support cartilage regrowth more difficult. Conversely, short picosecond (e.g., about 260-500 picosecond) plasma mediated ablation with sequential overlapping shot's will disrupt bone tissue and provided a much better clinical result as the shockwaves do the work without denaturing large amounts of tissue.

Sequential adjacent shots may employ a delay in time between shots such that the delay is selected to match the shockwave transit time between focal zones. Subsequent shots are delivered to add to the passing shockwave wave, enhancing pressure disrupted treated regions. Each fired shot adds to the previous shockwave as it passes.

The long term presence of macrophages in tissues previously treated by plasma bubbles in the maximum shockwave regime (300-500 picoseconds) will be an indicator of the utility of the "onion" shockwave and pressure wave wound wherein cell layers closest to the ablation bubble die rapidly, and more distant cell layers will die off more gradually. Macrophages actively phagocytizing dead cell material for an extended duration is expected to enhance tissue healing. A regime of creating injuries through shockwaves and pressure waves provides a new mechanism to treat tissue and to create optimally tuned plasma bubble (ablation bubble or cavitation bubble) expansion rates for the creation of novel shockwave and pressure wave wounds.

Shockwave and pressure wave focusing.

A phased array method may be employed to focus shockwave and pressure wave injuries more deeply into tissue. Several approaches may be employed for placement of LIOB injuries with micro-lens arrays.

Micro lens parabolic configuration:

Sections of micro lens arrays could contain one or more lens such as one or more parabolic shaped lenses 215. For example, referring to Figures 2A and 2B, in one embodiment "four lens" parabolic lens clusters 212 are at the tissue surface 230 and are focused on the same deep spot 220 within the tissue, which is useful to minimize fluence through the intervening tissue until co-incident at the "deep spot" target area 220. Here, the breakdown threshold for LIOB is only achieved where the irradiation overlaps at spot 220. There could be multiple, for example, hundreds or thousands of parabolic lens clusters (e.g., thousands of four lens parabolic lens clusters 212) in a micro-lens array. Use of lens clusters can prevent unwanted LIOB in the intervening shallower tissue while still provides high enough fluence to initiate LIOB in the relatively deeper target area 220.

An array of single lenses and a quasi-parabolic quad cell micro lens array in tissue:

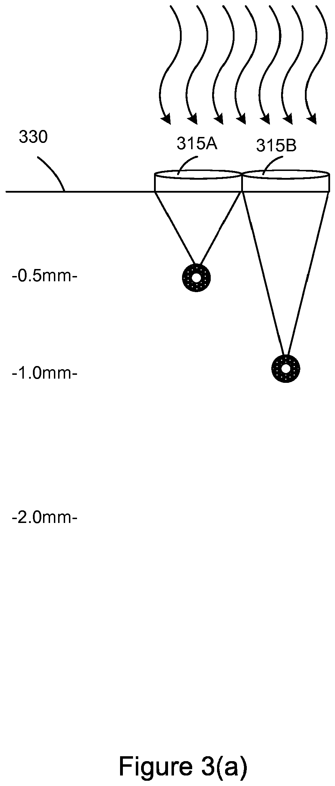

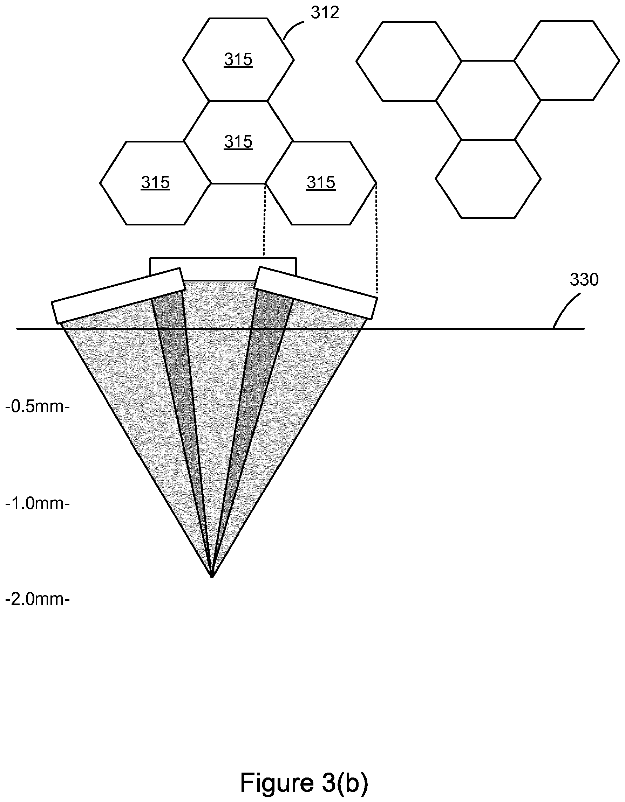

Figure 3A shows a two single lenses 315A and 315B positioned as a skin surface 330. Each of lenses 315A and 315B has a single discrete focus point with 315B achieving a deeper focus depth than lens 315A under otherwise constant treatment conditions. The depths from the skin surface 330 that can be achieved with a single lens such as 315A and 315B in skin tissue using a alexandrite laser in the picosecond regime is limited due to scattering within the tissue. For example, the depths of about 0.5 mm and about 1.0 mm can be achieved with single lenses 315A and 315B respectively. To achieve a deeper depth with a single lens would require an increased fluence that is undesirable for other reasons including that laser light scatters in tissue (due to photon scattering) thus, to effect sufficient fluence to achieve breakdown in the desired deeper area therefore the intervening shallower tissue would necessarily be exposed to even greater fluence than the targeted deeper areas. This would trigger the formation of lesions more shallow than the desired target depth. To address this limitation, Figure 3B shows a quasi-parabolic quad cell micro lens array 312 that can be employed to reach a relatively deeper depth (e.g., at a depth of about 2 mm) from the tissue surface 330 when using for example an alexandrite laser in the picosecond regime. The use for such a multi-cell array (e.g., quad cell micro lens array 312) can enable enhanced depth to be achieved at reduced fluence level than would be required if a single lens were employed. The use of a multi-cell array such as a quad cell micro lens array 312 enables enhanced depth that reaches a deep spot target area 320 through tissue to while avoiding shallower than desired LIOB. Referring still to Figure 3B, the lens cluster 312 including parabolic-like shaped micro lenses 315 overlaps focal points from 4 micro-lenses 315 onto a single deeper target area 320, which measures about 2.0 mm deep from the tissue surface 330.

Use of different lenses to target varying depths in a tissue region:

Relatively shallower targets can be adequately addressed, for example, using single cell micro-lenses, or fewer cell micro-lenses, for example. A single tissue area can have relatively shallow targets and relatively deep target and a combination of lenses (e.g., single cell micro-lenses and multi-cell micro-lenses such as quad-cell micro-lenses) can be employed to address the entire depth of the tissue to be treated. Use of one or more of the embodiments disclosed in association with Figures 3(a)-3(g) can be employed, for example, to treat scars.

Deep Treatment Pass:

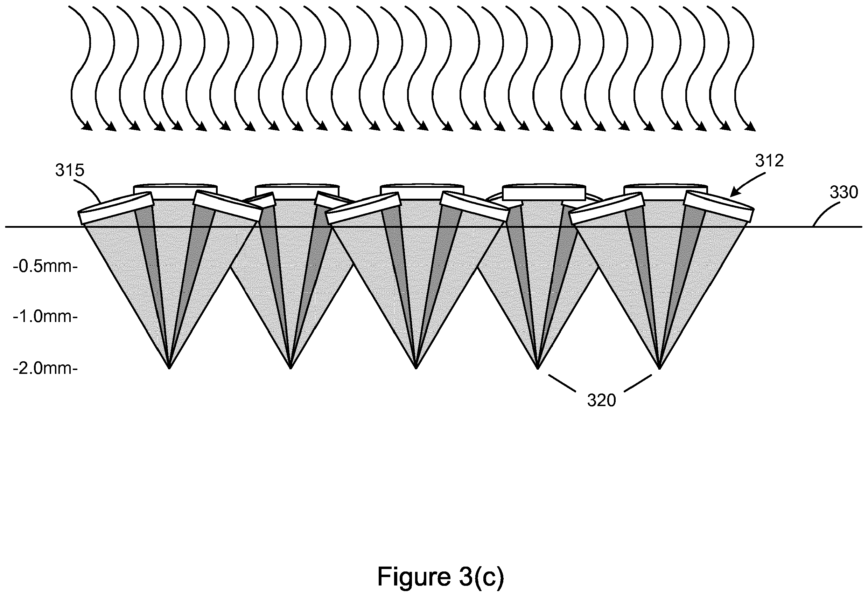

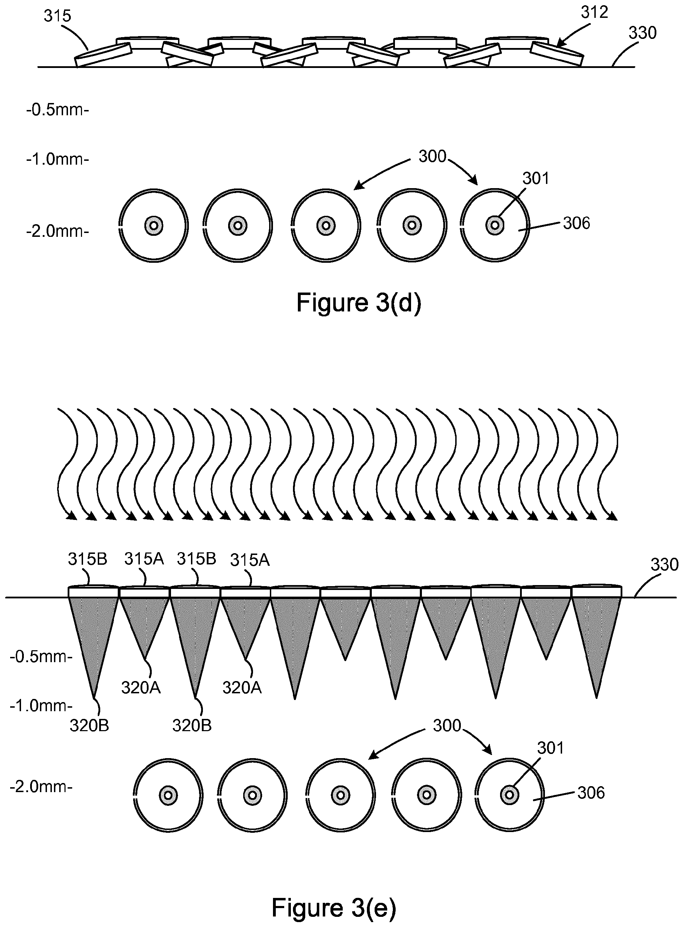

Figure 3(C) shows a plurality of lens clusters 312 including quad cell (e.g., 4 micro-lens) deep focus micro-lens arrays 315. Each 4 micro-lens array targets a relatively deep spot treatment area 320 thereby creating deeper LIOB lesions (e.g., at about 2 mm depth from the tissue surface 330) than would be possible if single cell micro lens arrays (or fewer cell micro lens arrays) were employed. The injury created in the spot treatment area(s) 320 shown in Figure 3(c) are shown in Figure 3(d) in which the injury 300 includes both an ablation lesion 301 and mechanically damaged regions 306 of tissue (region 306 includes tissue that is subjected to shock waves and/or pressure waves) that are created by the lens clusters 312 of deep focusing micro-lens arrays 315.

Shallow & Medium Treatment Pass:

Figure 3(e) shows a plurality of single cell focus micro-lens arrays 315A and 315B that create two distinct layers of LIOB lesions in a second pass. One LIOB lesion layer is shallower than the other (e.g., spot treatment areas 320A are shallower than spot treatment areas 320B). More specifically, treatment areas 320A at a depth of 0.5 mm from the tissue surface 330 and correspond to focus micro-lens array 315A and spot treatment areas 320B are at a depth of 1.0 mm from the tissue surface 330 and correspond to focus micro-lens array 315B. Both treatment area lesions are shallower than the layer of LIOB lesions created by the clusters 312 in Figures 3(c) and 3(d).

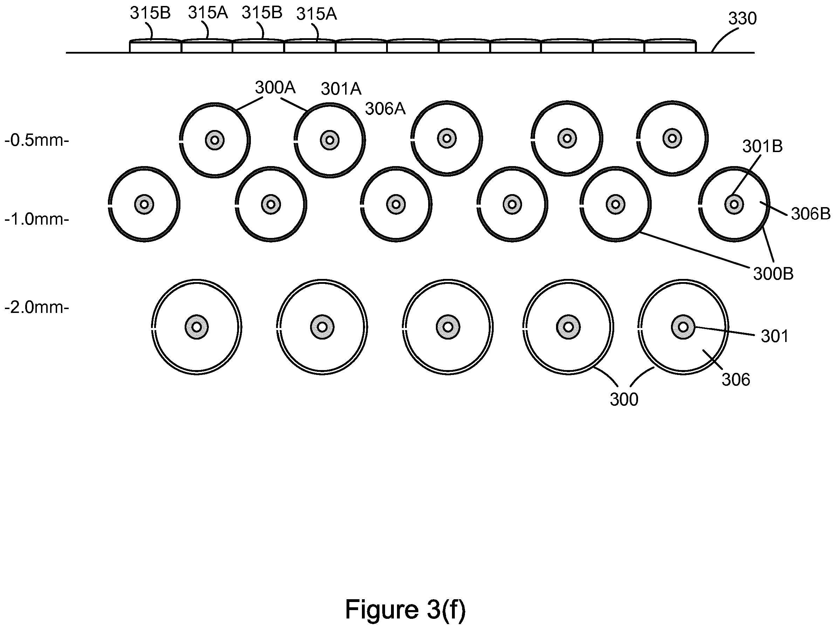

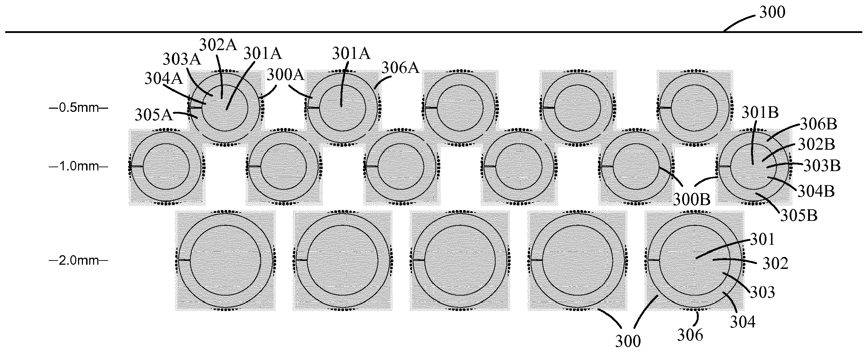

Figure 3(f) depicts the shallowest layer of tissue injury 300A including cavitation bubble 301A and the associated mechanically damaged region 306A of tissue which is at a depth of about 0.5 mm below the tissue surface 330. It also depicts the next deepest layer of tissue injury 300B including cavitation bubble 301B and the associated mechanically damaged region 306B of tissue which is at a depth of about 1.0 mm below the tissue surface 330. And finally it depicts the deepest layer of tissue injury 300 including cavitation bubble 301 and the associated mechanically damaged region 306 of tissue which is at a depth of about 2 mm below the tissue surface 330.

Figure 3(g) provides another more detailed depiction of the various depths of tissue treatment discussed in association with Figures 3(a)-3(e) in which layers of depth of LIOB lesions are formed in tissue treated with a 2-Pass treatment. Onion-like mechanical injuries 306A, 306B, and 306 (e.g., shockwave and pressure wave injuries) are disposed around an ablation/cavitation bubble cavity 301A, 301B, and 301 that is within their center. The first layer adjacent the cavitation/ablation bubble cavity center 302A, 302B, and 302 has severe mechanical cell damage (cause by shockwaves), and the subsequent layers away from the bubble cavity center have lessening cell damage with the outmost layer(s) (e.g., 304, 305A, and 305B) having relatively minor cell damage caused by pressure waves. Figure 3(g) is illustrative and the various onion-like layers of these injuries are not necessarily progressing at the same time.

Phased array approach:

A phased array approach times the delivery of a plurality of LIOB injuries such that shockwave-front is shaped to converge on a single deeper target area. A picosecond wavelength source 433 is impinged on a phased lens array. Referring now to Figure 4(a), an array of lenses 417A-417F are located and/or selected such that they are impinged by a shockwave 433. The lenses (e.g., 417A, 417B, 417E, and 417F) positioned at the outer edges of the desired shockwave 433 are initiated first (via LIOB's) and lenses closer to the center of the desired wavefront (e.g., 417C and 417D) are initiated later. Here distinct lenses 417A-417F are positioned and/or selected so that the LIOB's can be carefully timed to "shape" a composite wavefront 443, which can be driven by the LIOB's that impinge on lenses 417A-417F to provide a greater range of treatment depth and/or a greater magnitude shockwave fronts toward the shockwave target 420.

Referring also to Figures 4(b) and 4(c), in one embodiment, all LIOB's are focused on a single plane called the plane of LIOB focus 441 yet the arrival time of the injuries are selected and/or manipulated to shape the resulting shockwaves into a desired focus (e.g., to focus at a shockwave target). Figures 4(b) and 4(c) show the time delay of LIOB initiation being selected and/or manipulated such that 445B is the time delay of initiation of LIOB "B" provided by lens 417B that is less delayed than the time delay 445C that is a relatively longer time delay of LIOB initiation of LIOB "C" provided by lens 417C. Referring to Figure 4(c) this Quasi-Phased Array technique for collective LIOB shockwave shaping of a composite wavefront 443 can optimize the shockwave effect to create shaped wavefronts from a plurality of precisely timed LIOB injuries (e.g., injuries A-F). This is likely applicable to tissue types most susceptible to shockwaves.

In one embodiment, the user selects one lens suited for time delay (e.g., lenses 417B and 417E) and a different lens suited for a longer time delay (e.g., lenses 417C and 417D). Lens thickness may be selected to delay and/or accelerate the timing of the treatment. Referring still to Figures 4(b) and 4(c), since lenses 417A and 417F are "fired" first, they travel farther before becoming co-incident with the desired wave front 443 shape. The wave front 443 shape may be adjusted by introducing additional propagation delay (or less propagation delay) at lenses where additional delay or less delay is desired.

Sequential one two pulse:

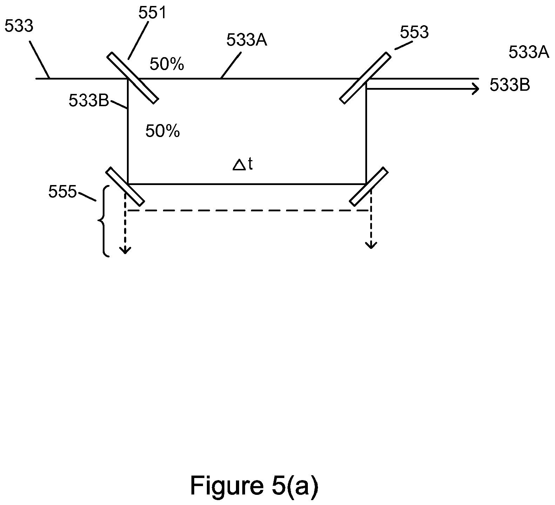

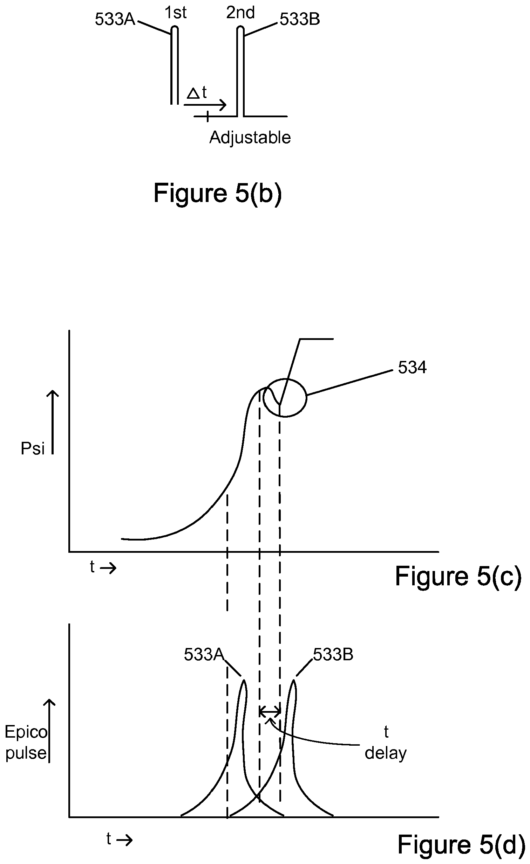

Referring now to Figures 5(a)-5(b), in a sequential one two pulse arrangement a picosecond drive pulse 533 is split by an adjustable beam splitter 551 into two equal parts 533A and 533B (e.g., 50% in the first part 533A and 50% in the second part 533B). One part (e.g., the second part 533B) can be delayed by an adjustable amount of time, therefore the first pulse 533A initiates LIOB (LIOB is not shown in this graphic) and the second pulse 533B arrives at the target while the target area is still ionized by the first pulse 533A, which previously initiated the LIOB. A second beam director 553 such as a beam splitter can also be use to further direct light as shown. Here, due at least in part to the delay, the second pulse 533B is immediately and fully absorbed by the target area and acts to drive a second pulse of expansion. The amount of delay between pulse 533A and 533B, shown as $\Delta T$, can be adjusted by moving 555, which is a reflective or substantially reflective surface such a mirror.

Referring now to Figures 5(c)-5(d), in one embodiment there is a point where the peak pressure provided by the first pulse is just beginning to wane (but is still in a plasma/ionized state) then consequently the initial shockwaves generated by the first pulse will detach (such that it is no longer driven by the ablation bubble expansion) and will begin to propagate into the tissue just as the second pulse arrives, this is depicted in Figure 5(c) as point 534 in a plot shown the time on the x-axis and the pressure in Psi on the y-axis. It is believed that this shockwave detachment will result in the second shockwave overtaking the initial shockwave wave front thereby providing an additive re-enforcement of the initial shockwave extending the range and the volume of pressure injured tissue.

Thus, the first LIOB is formed by a first pulse 533A and before it de-ionizes the LIOB is further driven to a second period of bubble expansion. This creates a second shockwave pulse 533B that, if sufficiently driven, can overtake and add to the first shockwave. The delayed second shockwave of the sequential one two pulse arrangement extends the volume of tissue treated with efficacious shockwaves. Applicants believe that the second pulse of energy benefit is larger than the first pulse, because the second shockwave pulse can catch up to and add to the first pulse wave front.

It is possible to adjust the energy provided in the first vs the second pulse (e.g., 533A vs 533B via the beam splitter) to tune the secondary wave front optimization. For example, first pulse may have less energy than the second pulse to support optimum wave front overtaking and additive pressure effects. In one embodiment, the one-two sequential firing can provide overlapping shots of the same target area. In another embodiment, the one-two sequential firing can have two adjacent target areas, for example.

A sequential one two pulse technique can provide an enhanced lesion. For example, a sequential one two pulse technique can optimize the shockwave effect by delivering a $2^{nd}$ laser pulse to the target (LIOB expanded bubble) before ionization and non-linear absorption has discontinued. The technique initiates a second expanding shockwave to increase lesion size. Figures 5(a)-5(d) illustrate this approach. This is especially useful when delivering LIOB injuries as deeply as possible. This method allows for large ablation volumes, while keeping fluence through intervening tissue as low as possible (50/50 energy in shot 1 (e.g., 533A) and in shot 2 (e.g., 533B)).

In some embodiments, this sequential one-two pulse technique is paired with the quasi-parabolic 4 cell micro-lens array disclosed in association with Figures 2(a)-2(b) and 3(a)-3(c), for example, to achieve a deeper reach, and is used as a method to increase ablated volume without increasing single pulse energy. Generally, ablated volume is proportionate to laser energy/pulse and this sequential one two pulse technique can achieve greater relative ablation volumes without increasing the applied energy than in the absence of using the sequential one two pulse technique. Suitable applications of the one two pulse technique alone and the quasi-parabolic 4 cell micro-lens array used alone or in combination can include treatment areas where a deep but also large lesion is desired.

LIOB energy expression- shock wave energy/pressure wave energy vs. thermal energy

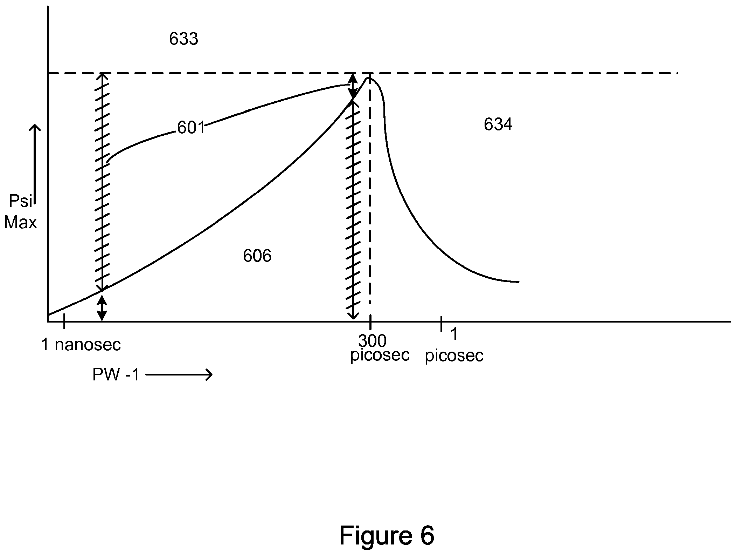

A laser pulse drives LIOB. Applicants believe that the pulse width determines whether the pulse energy manifests as principally shock wave/pressure wave energy, principally thermal energy or a mix of pressure wave energy (shock wave energy) and thermal energy. Figure 6 depicts the generalized relationship between thermal energy 601 and pressure wave energy 606 (shock wave energy) measured in psi as a function of pulse width. When the pulse width is in the nanosecond regime (e.g., 1 nanosecond) there is a large thermal energy 601 effect and a small pressure wave energy effect 606. When the pulse width is in the picosecond regime (e.g., about 300 picoseconds) there is a large pressure wave energy effect 606 and a small thermal wave energy effect 601.

Picosecond LIOB with a fractional beam array

Use of a picosecond laser with an output beam modified via a fractional array (e.g., a micro-lens array that creates high intensity focal zones surrounded by non-treated or less treated area of tissue) or a non-uniform beam characterized by a cross-section corresponding to an array of relatively small, relatively high-fluence, spaced-apart regions superimposed on a relatively large, and relatively lower-fluence background provides thermal energy and mechanical energy that cause thermal injury and shockwave and/or pressure wave injury. Where the picosecond laser with the non-uniform array treats tissue there is a component of high fluence causing thermal damage. The regime of injury caused by heat is well known.

In contrast, the regime of injury effected by the combination of thermal energy and mechanical energy provided by the shockwaves and pressure waves resulting from treating tissue with a picosecond laser such as a PicoPulse™ laser with CAPS™ technology is new and is not yet well defined, however, applicant believes it is desirable to understand the effect on the tissue of these combined thermal and mechanical effects. The energies may happen at a different rate and at a varied combination. The balance of the thermal and/or mechanical energies may be controlled to achieve a desired tissue interaction/tissue effect.

Samples of tissue treated with a picopulse laser with a non-uniform array reviewed 3 months after treatment showed some elongation of elastic fibers. Under prior regimes for tissue treatment elastin elongation is not typically seen without a lot of thermal injury that leads to a great deal of downtime. Subject downtime limits treatment application to a relatively smaller group of subjects willing and able to devote time to recovery due to the obviousness of their treatment or to a smaller number of tissue sites that may be covered during the obvious recovery. Elastin elongation with minor to no downtime is surprising and desirable in that tissue treatment that leads to desired elastin elongation with less to no downtime opens up the treatment application to the larger population that can't afford downtime and to otherwise open tissue sites where obviousness signs of treatment dissuade treatment of the area.

It is understood in the prior art that in order to damage elastin a large thermal injury and/or a high temperature/fluence was required. Treatment with a picosecond laser was applied using a non-uniform beam array to achieve very high temperatures locally in a small area, this local thermal energy was combined with mechanical energy (e.g., shock wave and/or pressure wave energy).

Without being bound to any single theory Applicant's believe that the elastin elongation is a result of (a) intense thermal action in a small area, (b) mechanical energy (shockwave and pressure wave energy) of the LIOB or (c) a combination of finite thermal intensity and mechanical energy of the LIOB.

In one embodiment, the picopulse laser is at 755 nm and is absorbed in melanin and hemoglobin. A purpura like effect was observed upon use of a picopulse laser on tissue. Large voids are not observed in the treated tissue. The red blood cells are dispersed throughout the tissue and the temporal dimension of the red blood cells is larger (e.g., at a depth) than that of melanin which is in a local area of the tissue. The dispersal of the red blood cells throughout the tissue could magnify the mechanical part of the effect, which due to the hemoglobin being a target of the 755 nm wavelength, occurs throughout the tissue at the location of the red blood cells in the target. The red blood cells provide an absorptive target which, at sufficient fluence, may breakdown and serve as the LIOB center. High temperatures imparted to the target red blood cells by the treatment are substantially confined within the ablation volume whereas outside the ablation volume of the bubble the rapid LIOB expansion coverts energy to shock and/or pressure waves (mechanical effect).

An Elastin Stain (known as an EVG stain) to study the impact of the picopulse with focused treatment regions of tissue separated by untreated regions of tissue (e.g., a CAPS array) on elastin right after tissue treatment and then later one or more months after tissue treatment, for example, three months after treatment can be performed. The EVG stain looks for the live elastin cells. Once elastin cells are thermally denatured they cannot be seen via EVG stain. If elastin fibers are mechanically broken due to the impact of pressure then the EVG stain provides a different result that indicates the mechanical damage to the elastin fibers. It is expected that the EVG stain will indicate that elastin fibers (and cell contents) principally damaged by shockwaves will still be visible by means of EVP stain whereas elastin cells damaged by thermal effect will be denatured and thus invisible by means of EVP stain. This effect can distinguish whether thermal or shockwave effects predominantly mediated the tissue injury.

Scar tissue tends to have fewer and/or no elastin fibers compared to non-scarred tissue (e.g., non-scarred skin tissue). It is believed that treatment of scarred tissue with the picopulse laser (with or without CAPs technology) stimulates the scarred tissue to enable the elastic fibers (e.g., elastin fiber) to rebuild in the location of the scar tissue. Use of the picopulse laser regime is believed to reprogram the scar tissue to increase elastic fiber content and to enable the scarred tissue to heal itself by releasing elastic fibers thereby enabling the tissue to appear and/or become more like normal non-scarred tissue.

Controlled re-programming of the scar, of the composition of the scar, specifically its elastic fiber content, is important. It is believed that reforming elastin in the tissue may be controlled by:

A) Tissue treatment using a wavelength of 755 nm and at a pulse width duration between about 500 picoseconds and about 750 picoseconds has been seen to positively affect the elastin elongation (e.g., lengthen and thicken elastin content in the treated tissue). Elastin elongation is not seen using a similar wavelength (e.g., about 755 nm) at nanosecond pulse width durations (e.g., pulse width durations of about 3 nanoseconds or greater). This indicates that an ideal injury that promotes elastic fiber elongation has or includes a mechanical component (e.g., shock wave and/or pressure wave induced or is induced by a combination of a thermal injury and a mechanical injury) as opposed to being only thermally mediated. It is believed that the optimum pulse duration for maximizing elastin elongation will coincide with the duration that provides the maximum shockwave treated volume, observed to be about 260-300 picoseconds.

B) Controlling the fluence for generating picosecond LIOB micro-injury: Firstly it is necessary to exceed the ablation threshold for the target area and this is absorption dependent. One needs to instantaneously ionize the atoms in the target cell to initiate LIOB. Fluences of between about $0.08 \text{ J/cm}^2$ and about $50 \text{ J/cm}^2$ will at least exceed the ablation threshold for most biologic tissue constituents. Suitable fluence ranges that may be employed include, for example, about $0.08 \text{ J/cm}^2$ or greater, from about $0.08 \text{ J/cm}^2$ to about $50 \text{ J/cm}^2$, from about $0.08 \text{ J/cm}^2$ to about $5 \text{ J/cm}^2$, about $0.08 \text{ J/cm}^2$ to about $20 \text{ J/cm}^2$. Secondly, fluence ranges greater than the ablation threshold are absorbed by the LIOB bubble and act to drive further bubble expansion. Accordingly, an optimum fluence is a fluence that is at least above the ablation threshold for the target area. In addition, greater fluences can be selected to control the resulting volume of the ablation bubble. The minimum fluence that triggers LIOB if exceeded can convey a larger thermal injury volume, but not necessarily a larger volume of shock wave and/or pressure wave than if the minimum fluence triggered LIOB.

C) Controlling the wavelength. Wavelength and fluence matter because they depend on linear (e.g., normal) absorption to enable LIOB formation. The wavelength can be selected in part to enable a target chromophore to be the site of the LIOB. For example, if you want to be specific for location you can combine the wavelength selection optionally together with focusing. One can choose a wavelength to allow penetration to assure LIOB forms at the desired depth. If you want a shallow treatment one can provide a shallow focus. For example, 1064 nm is weekly absorbed so one can determine the injury cite primarily by employing the appropriate amount of focus to achieve the desired depth of LIOB. The wavelength of 755 nm is appropriate for targeting blood. Because 755 nm is a strongly absorbed wavelength, focusing at this wavelength is relatively challenging, because the absorbing chromophore 755 nm will likely initiate an LIOB lesion prior to (shallower than) the desired depth. One might choose a wavelength that has some linear absorption that can dictate how deep into the tissue one can focus. Thus, formation of LIOB's at greater depths in tissue is best accomplished by selection of a weakly absorbed wavelength such that LIOB formation occurs at the relatively "deep" focal point, where the fluence exceeds the LIOB threshold as opposed to occurring at a shallower depth when a more readily absorbed wavelength contacts a highly absorptive chromophore.

D) Apportioning how much injury is mechanical (shock wave and/or pressure wave) versus thermal. Without being bound to a single theory, Applicants believe that mechanical damage may favor elastic fiber elongation so treatment can be controlled to favor mechanical damage. For example, where greater photomechanical damage compared to photothermal damage is desired employing a picosecond pulse is better. When a shorter pulse width is used the single peak shock wave pressure front gets higher than, at a certain point, the single peak pressure font drops. The means that the picosecond range can offer greater mechanical damage then thermal damage than in other regimes.

E) In some embodiments, a combination of thermal and mechanical injury is desired. For example, one can provide a combination of picosecond and femtosecond damage at to tissue such that at the molecular level both thermal and mechanical breakdown are provided.

F) A pulse width range where photomechanical (damage or energy) and photothermal (damage or energy) effects are provided can impact differing cell types according to the cells susceptibility to the applied energy whether mechanical (e.g., shockwave and/or pressure wave) or thermal. The radius of effect whether shockwave and/or pressure wave or thermal and the range of susceptible cell types within that range can be complex. When considering the selection of an optimum pulse duration between for example about 50-100 nanoseconds to about 260-300 picoseconds, where durations between about 260 to about 900 picoseconds will deliver predominantly shockwave energy and where durations above about 900 picoseconds or above about 1000 picoseconds will deliver predominantly thermal energy to the tissue region proximal to the ablation bubble. Durations in the middle of the range generate both shockwaves and thermal effects, which may be useful to create a blend of pressure injury and thermal injury. Assuming roughly equivalent laser pulse energies and beam quality, durations around about 260-300 picoseconds are expected to provide an improved duration for generation of maximum magnitude shockwaves that will treat the largest volume of tissue with mechanical waves (e.g., shock waves and/or pressure waves). Conversely, selection of the longest duration in the available range of about 50-100 nanoseconds, provide an improved volume of thermal injury proximal to the ablation bubble. Therefore if the clinical goal is maximized shockwave and/or pressure injury volume then a laser pulse duration of 260-300 picoseconds is preferred. If the clinical goal is to maximize thermal injury volume then the laser pulse duration of about 50-100 nanoseconds could be selected.

The treatment of tissue using a light source (e.g., laser) in the regime of pulse duration in the picosecond range (without or without CAPs focusing) can elicit a mechanical injury healing process, or a combination mechanical injury and thermal injury healing process and the presence of mechanical injury healing can lead to desirable effects, including: The range of photothermal to mechanical effects (with or without CAPs focusing) can be adjusted to optimally treats scars, pigment, skin wrinkles, and skin laxity. The pulse width range of about 260-300 picoseconds is believed to make the greatest volume of pressure injury while simultaneously minimizing the deposition of extra-ablation bubble thermal energy. Pressure injury is believed to promote elastic fiber elongation. A method of controlling the ratio of mechanical to thermal damage effect can be accomplished by selecting a pulse width for the purpose of controlling the injury including promoting elastic fiber elongation and/or to optimize healing and/or new cell types expressed. Such control can lead to better treatment outcomes.

An embodiment of this includes a system that allows for changing the ratio of mechanical (e.g., pressure wave or shock wave) to thermal effect depending on the desired target treatment. This way a single system can be employed to treat one indication (e.g., scars, pigment, wrinkles, laxity) using a pulse width that provides a first ratio of mechanical to thermal effect and then by tuning the controller to a different pulse width that provides a different ratio of mechanical to thermal effect that can be employed to treat another indication (e.g., scars, pigment, wrinkles, laxity).

In one embodiment, a system that improves tissue due to a combined mechanical and thermal effect has a pulse range of from about 150 picoseconds to about 900 picoseconds and has a relatively low fluence (e.g., from about 0.08 J/cm$^2$ to about 2 J/cm$^2$)

Peak energy densities of between about 2 to about 50J/cm$^2$ will exceed the ablation threshold in a collagen gel and in a porcine cornea (Oraevsky et al fig 4) somewhat representative biological tissues. In the picosecond range therefore, fluences of between about 0.08 J/cm$^2$ about about 50 J/cm$^2$ will at least exceed the ablation threshold for most biologic tissue constituents. Generally, a fluence range of from about 0.08J/cm$^2$ to about 2 J/cm$^2$ can be employed for highly absorbing targets an up to about 50J/cm$^2$ (e.g., from about 3 J/cm$^2$ to about 50 J/cm$^2$) for weakly absorbing

targets.

A fractional tissue treatment employing a pulse width, e.g., from about 150 picoseconds to about 900 picoseconds, or from about 260 picoseconds to about 300 picoseconds causes a pressure wave treatment (e.g., a picopulse fractional treatment) by providing a fractions of ablated volume and prevents thermal injury outside of the small fractions of ablated volumes and instead treats tissue outside of the ablated volume mechanical energy (e.g.,with shock waves and/or pressures waves). Generally, the ablation volume and the resulting mechanical waves (e.g., pressure waves and/or shock waves) are disposed below the surface of the tissue, at a depth. In contrast, photo thermal fractional treatment such as is available utilizing a Palomar 1540 fractional laser thermally denatures fractions of tissue that are surrounded by untreated or less treated tissue without treating the tissue surrounding the thermally denatured fractions with any mechanical energy (e.g., without any shock wave or pressure wave adjacent the thermally denatured fractions).

The applicant has surprisingly discovered that applying a light based system (e.g., a laser system) with or without a fractionated beam profile at the unique pulse width of from about 150 picoseconds to about 900 picoseconds, from about 200 picoseconds to about 500 picoseconds, or from about 260 picoseconds to about 300 picoseconds to tissue first imparts a photothermal injury and from that photothermal injury emanate mechanical waves (e.g., shock waves and/or pressure waves) that injure cells and tissues in a manner that stimulates cells and/or causes tissue rejuvenation. The injury created by shock waves and/or pressure waves appears to be well suited to tissue rejuvenation. In particular pressure wave and/or shockwave tissue damage is suited to treating the skin (e.g., for pigmented lesions, scars, laxity, wrinkles, stria), lungs (e.g., for asthma, chronic obstructive pulmonary disease (COPD), damage related to smoking (e.g., smoking tobacco), and liver disease including cirrhosis).

We appreciate that it may be advantageous to tune the injury to contain both thermal and shockwave aspects to tailor the injury and the consequent outcome. For example, when repairing a stiffer tissue such as the bottom of the feet you might use a pulse width that provides more thermal injury to provide a stiffer character to the skin. For example, if treating a softer tissue such as a part of the face you might use a pulse width that provides more of a mechanical injury (e.g., a shock wave or pressure wave injury) to provide a softer character to the skin.

Optionally, one can further tailor the injury to provide a deeper thermal injury to provide a stiffer character that acts as scaffolding for example for a non-invasive face lift effect and/or for facial reconstruction when for example ligaments have been damaged or lost. A shallow shockwave can be employed to soften the character of skin on top of the thermally treated tissue. A tuned treatment could have a first pass with a first pulse width in the nanosecond range that goes deep and a second pass with a second pulse width in the picosecond range that goes shallow. A tuned treatment can be employed to kill nerves in painful scars including, for example, hypertrophic scars. Systems employed for such tuned treatments may have a single beam, a non-uniform beam, and/or a fractional beam.

The lower pulse width in the picopulse range matters because it results in shock waves that occur above the LIOB threshold near regions of high absorption resulting in LIOB breakdown. The bubble expansion drives a steep edged high pressure wave front that appears to be very useful for causing larger injuries (micro lesions). As the wave front attenuates it transitions to sub-sonic propagation and becomes a "pressure wave" thereafter. Pressure waves occur below the LIOB threshold near regions of high absorption. The high pressure recoiling pressure waves are sufficient to cause injury (micro lesions) to some radius of the absorption center.

As discussed herein, LIOB's can be tuned for the purpose of generating maximal shockwaves and pressures. Two primary ionization processes drive LIOB's. These processes are:

(1) Multiphoton ionization, which occurs at very short tens of picosecond pulse widths to femtosecond pulse widths. This pulse width range confers colorblindness with zero absorption required for LIOB. This range is ideal for opthamology, because it has high ablation efficiency. When multiphoton ionization is in the femtosecond range it expands the LIOB bubble at a fast rate, with such a high ablation efficiency such that it consumes its own shockwave. Thus as pulse widths get shorter, below about 220 picoseconds (e.g., about 220 picoseconds or less, or about 210 picoseconds or less) multiphoton ionization takes over and the shockwave magnitude is reduced. The mutiphoton ionization process below about 220 picoseconds results in an increased target tissue temperature. The governing process may be described as photothermolysis. The method of action is via a change in target temperature that increases the temperature of the target. The pulsewidth selection whether in the nanosecond or in the picosecond regime (e.g., about 220 picoseconds and below) should be driven by the desired thermal effect and the wavelength should be selected for a particular chromophore color.

(2) Electron avalanche, which occurs in regions of high fluence and absorption in the high hundreds picoseconds (e.g., from about 260 picoseconds to about 900 picoseconds) is believed to have a mechanism of action that increases target pressure and may initiate secondary shockwaves. The pulse width range between about 260 picoseconds and about 900 picoseconds, or about 260 picoseconds to about 300 picoseconds is ideal for shockwave development, because at pulsewidths shorter than about 260 picoseconds multiphoton ionization, a much faster process than electron ionization, begins. Below about 260 picoseconds and into the femtosecond range the ablation efficiency improves so much that all of the laser pulse energy is consumed driving the expansion of the LIOB bubble leaving relatively no energy remaining to drive pressure or shockwaves. The governing process may be described as photobarolysis due to pressure/shockwaves. The method of action is via a change in target pressure that increases the pressure in the target and optionally introduces shearing stresses. The pulsewidth selection in the picosecond regime should be drive by the desired mechanical effect and the wavelength should be selected for a particular chromophore color.

Anticipated histology:

It was expected that the tissue and cells that experience immediate cell death and cell damage that leads to eventual cell death (see, e.g., Figure 1 "the onion") and yield new cell stimulation and/or cell repair. The new and/or repaired cells are expected to include erythrocytes and macrophages that lead to the formation of new collagen and new elastin, for example.

Histology Evaluation Protocol 1: Inflammatory Response in the Dermis Indicating mechanical damage indicative of LIOB

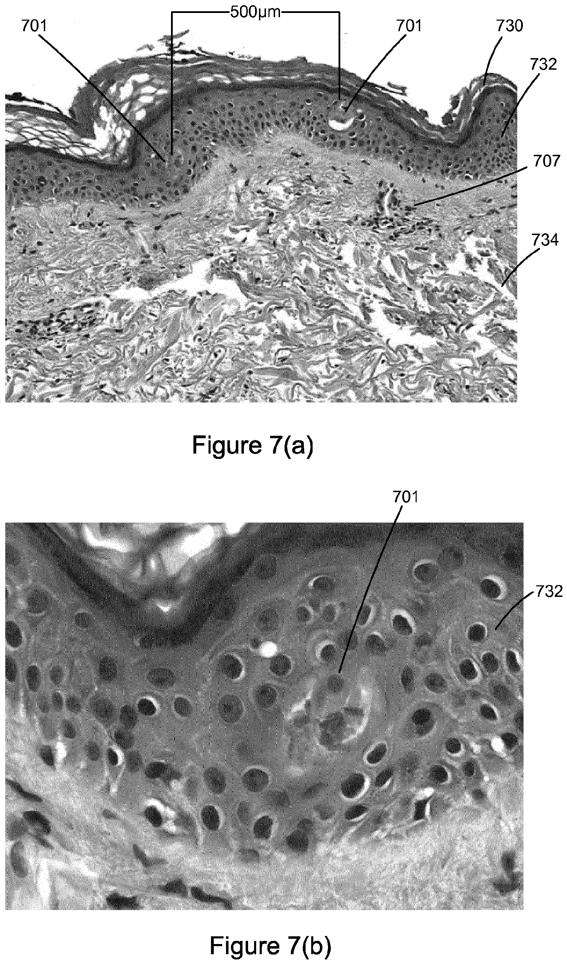

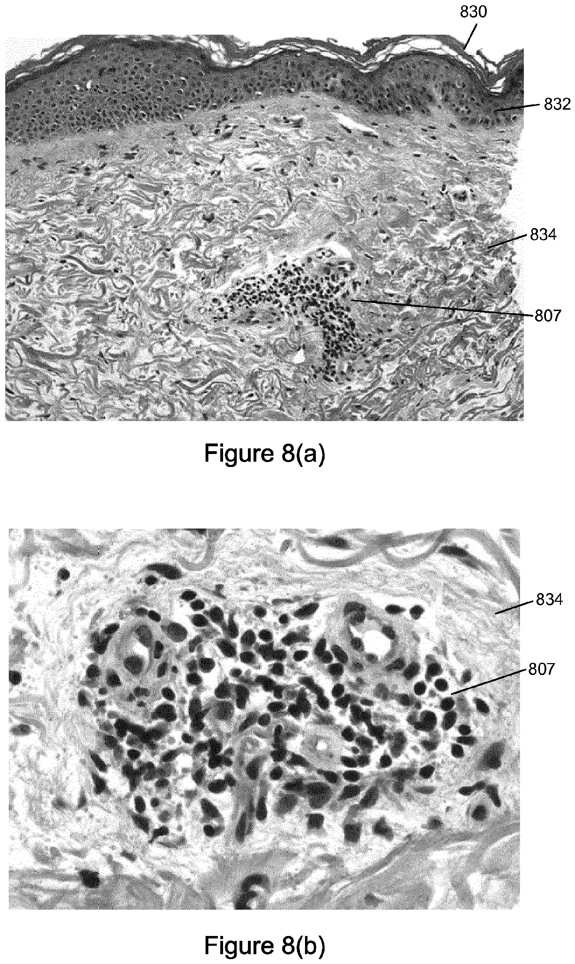

A PICOSURE® picosecond laser having a 755 nm wavelength utilizing a lens array having the FOCUS™ trade name with a 6 mm spot size, at a fluence of 0.71 J/cm$^2$, and producing a pulse energy of 200 mJ, at a pulse duration of 750 picoseconds was utilized to treat Caucasian skin type II. The lens array provides a distance of about 500 $\mu$m between the center of adjacent lenses. At 24 hours post treatment a skin biopsy punch was taken and its histology was evaluated. A standard H&E Stain was utilized to evaluate the biopsied skin. Figures 7(a), 7(b), 8(a) and 8(b) show images of these biopsies. The damage bubbles 701 present in the epidermis 732 are shown in Figure 7(a) at 100 times magnification and in Figure 7(b) at 400 times magnification. Figure 7(a) shows that the damage bubbles 701 are spaced about 500 $\mu$m apart. Referring still to Figure 7(a) below the epidermis 732 in the dermis 734 there is no evidence of thermal coagulation in the dermis 734 or in vessels within the dermis 734, however, there is evidence of an inflammatory response 707 in the dermis 734.

Likewise in Figure 8(a) (at 100 times magnification) and 8(a) (at 400 times magnification) there is no evidence of thermal coagulation in the dermis 834 or in vessels within the dermis 834, however, there is evidence of an inflammatory response 807 which falls within the dermis 834 and outside of the vascular structure. This histology suggests that there is inflammation in the dermis 834 despite there being no evidence of thermal coagulation in the dermis. The histology fails to show evidence of thermal damage in the dermis 834. Instead there is an inflammatory response 807 deeper in the dermis 834 for example greater than 100 microns below the dermal epidermal junction and because there is no evidence of thermal damage within the dermis 834 the inflammation 807 is not prompted by thermal activity.

Applicants believe that the presence of inflammation 707, 807 in the dermis 734, 834 that is not a product of thermal damage within the dermis 734, 834 supports Applicants theory regarding the presence of and impact cause by mechanical effects (e.g., shockwave effects and/or pressure wave effects) that are prompted by the damage bubbles (e.g., 701) present in the epidermis.

Histology Evaluation Protocol 2: Mechanical damage provides an increase in dermal elastic fiber density and elastic fiber elongation previously associated with results from aggressive thermal injury.

A PICOSURE® picosecond laser having a 755 nm wavelength utilizing a lens array having the FOCUS™ trade name with a 6 mm spot size, at a fluence of 0.71 J/cm2, and producing a pulse energy of 200 mJ, at a pulse duration of 750 picoseconds was utilized to treat Caucasian skin at the site of surgical scarring. Table 1 displays results from 7 patients who consented to histologic evaluation of their treated scar. Up to two 2mm punch biopsies were taken within the treatment area (the site of the scar) at baseline, 2.5 weeks post treatment, 1 month post treatment and 3 months post treatment. The histology was evaluated by a pathologist. The summary of an independent pathologist's findings is in Table 1 below.

Table 1

| Patient No. | INITIAL | 2.5 WEEKS | 1 MONTH | 3 MONTHS |
|---|---|---|---|---|
| | | | | |

(continued)

| Patient No. | INITIAL | 2.5 WEEKS | 1 MONTH | 3 MONTHS |
|---|---|---|---|---|
| 1 | EVG STAIN | Moderate increase of density and thickening of EF | Same as 2.5 weeks | Elongation of elastic fibers |
| | Collagen 3 | Slight increase | Same as 2.5 weeks | Same as 2.5 weeks |
| | Collagen 1 | No Change | No Change | No Change |
| | Colloidal iron | No Change | Moderate increase in scar | Moderate increase in scar |
| 2 | EVG STAIN | Slight increase of density and thickening | Elongation of elastic fibers | |
| | Collagen 3 | Moderate increase | Moderate increase | |
| | Collagen 1 | No Change | No Change | |
| | Colloidal iron | No Change | Moderate increase in scar | |
| 3 | EVG STAIN | Slight increase of density and thickening | Elongation of elastic fibers | No Change |
| | Collagen 3 | Moderate increase | No Change | Slight increase |
| | Collagen 1 | No Change | No Change | No Change |
| | Colloidal iron | Slight increase | Moderate increase | No Change |
| 4 | EVG STAIN | No Change | Moderate increase of density and thickening | Moderate increase of density and thickening and elongation of elastic fibers |
| | Collagen 3 | Slight increase | Slight increase | No Change |
| | Collagen 1 | No Change | No Change | No Change |
| | Colloidal iron | No Change | No Change | Moderate increase |
| 5 | EVG STAIN | Moderate increase of density and thickening | NOT DONE | Moderate increase of density and thickening |
| | Collagen 3 | Slight increase | NOT DONE | Moderate increase |
| | Collagen 1 | No Change | NOT DONE | No Change |
| | Colloidal iron | No Change | NOT DONE | Moderate increase |

(continued)

| Patient No. | INITIAL | 2.5 WEEKS | 1 MONTH | 3 MONTHS |
|---|---|---|---|---|
| 6 | EVG STAIN | No Change | Slight increase of density | Slight increase in density and elongation of elastic fibers |
| | Collagen 3 | Slight increase | No Change | No Change |
| | Collagen 1 | No Change | No Change | No Change |
| | Colloidal iron | No Change | No Change | No Change |
| 7 | EVG STAIN | Moderate increase of density and thickening | | |
| | Collagen 3 | Slight increase | Slight increase | |
| | Collagen 1 | No Change | No Change | |
| | Colloidal iron | Slight increase | No Change | |

The summary of changes in seven patients consistently show an increased density and thickness of elastic fibers and an increase in mucin from 2 weeks to 3 months out. In addition, all subjects showed an increase in Collagen 3 at 2 weeks post treatment.

The reviewing pathologist concluded that the elastic fiber in tissue increased in density and thickness and in many cases the elastic fiber appears to have elongated (e.g., they appear to be longer).

Increased density and thickness of elastic fibers and/or elongation of elastic fibers are positive signs of restoring normal skin elasticity in the scar tissue and thus reducing the appearance of the scar. In addition, the histology results verified that the laser did not create any additional safety concerns and the tissue healed normally after the treatment.

Summary of Understanding due to Histology Evaluations of Protocols 1 and 2

In the Histology Evaluation of Protocol 1 we observe that there is limited thermal injury in the epidermis and there is no thermal injury in the dermis, however, there is a level of inflammatory response in the dermis that Applicants believe is created by the mechanical effects (e.g., shock waves and/or pressure waves) of the picosecond laser. As a result of the picosecond treatment and as observed using the Histology Evaluation of Protocol 2 we observe that that elastic fiber density and thickening in the dermis increased and/or elastic fiber elongation in the dermis increased after the treatment (at times as early as 2.5 weeks, 1 month, and 3 months after treatment). This type of elastic fiber response has been previously observed in tissue histology studies after light based treatments, however, only in instances of relatively aggressive ablative treatment(s) (e.g., with an ablative fractional treatment or an ablative full surface treatment such as with a $CO_2$ laser) and relatively aggressive non-ablative treatment(s) (e.g., with non-ablative fractional treatments) that rely on thermal injury to the dermis. Further, such inflammatory response in the dermis have been previously seen in the site of a large burn scar.

Accordingly, the picosecond laser systems employed in accordance with the instant disclosure provide an inflammatory response in non-thermally treated tissue regions (e.g., the dermis) that is unexpected and surprising in view of prior methods of eliciting an inflammatory response to a targeted tissue treatment. While not being bound to any single theory, Applicants believe that the mechanical effects of the picosecond treatment (e.g., pressure wave effects and/or shockwave effects) illicit the inflammatory response that appears to cause the increase in in fiber density and increase in fiber thickening and/or elongation of elastic fibers in the dermis.

The aspects, embodiments, features, and examples of the invention are to be considered illustrative in all respects and are not intended to limit the invention, the scope of which is defined only by the claims.

The use of headings and sections in the application is not meant to limit the invention; each section can apply to any aspect, embodiment, or feature of the invention.

Throughout the application, where compositions are described as having, including, or comprising specific components, or where processes are described as having, including or comprising specific process steps, it is contemplated that compositions of the present teachings also consist essentially of, or consist of, the recited components, and that the processes of the present teachings also consist essentially of, or consist of, the recited process steps.

In the application, where an element or component is said to be included in and/or selected from a list of recited elements or components, it should be understood that the element or component can be any one of the recited elements or components and can be selected from a group consisting of two or more of the recited elements or components.

The use of the terms "include," "includes," "including," "have," "has," or "having" should be generally understood as open-ended and non-limiting unless specifically stated otherwise.

The use of the singular herein includes the plural (and vice versa) unless specifically stated otherwise. Moreover, the singular forms "a," "an," and "the" include plural forms unless the context clearly dictates otherwise. In addition, where the use of the term "about" is before a quantitative value, the present teachings also include the specific quantitative value itself, unless specifically stated otherwise.

It should be understood that the order of steps or order for performing certain actions is immaterial so long as the present teachings remain operable. Moreover, two or more steps or actions may be conducted simultaneously.

Where a range or list of values is provided, each intervening value between the upper and lower limits of that range or list of values is individually contemplated and is encompassed within the invention as if each value were specifically enumerated herein. In addition, smaller ranges between and including the upper and lower limits of a given range are contemplated and encompassed within the invention. The listing of exemplary values or ranges is not a disclaimer of other values or ranges between and including the upper and lower limits of a given range.

The invention is as defined in the appended claims as follows.

**Claims**

1. A system for tissue treatment, comprising:

   a picosecond laser configured to emit light of at least one wavelength;
   a fractional optical system comprising a lens array including a plurality of foci for concentrating a laser emission, from the picosecond laser, to targets in the epidermis wherein the targets absorb light of at least one wavelength, and
   the picosecond laser configured to emit a pulse width within the range of from about 260 picoseconds to about 900 picoseconds and a fluence range from about 0.8 J/cm$^2$ to about 50 J/cm$^2$, wherein the at least one wavelength is absorbed by melanin and concentrating the laser emission at the targets initiates an electron avalanche ionization at the targets to provide a pressure wave emission to tissue adjacent the targets.

2. The system of claim 1 wherein the targets comprise melanin.

3. The system of claim 1, wherein the pulse width ranges from about 260 picoseconds to about 500 picoseconds.

4. The system of claim 1, further comprising a controller for controlling pulse width.

5. The system of claim 4, wherein the controller controls the pulse width to provide a greater thermal effect at a relatively longer pulse width or wherein tuning the controller to a different pulse width changes the ratio of mechanical to thermal effect on the tissue adjacent the targets.

6. The system of claim 4, wherein the controller enables a shorter pulse width concentrated at a relatively shallow depth to the targets and another relatively longer pulse width at a relatively deeper depth to subsequent targets; and/or

   wherein the controller enables one pass of the laser with the at least one foci concentrated at a first depth and another pass of the laser with the at least one foci concentrated at a second depth different from the first depth; and/or
   wherein the controller enables a first pulse of the laser and a second pulse of the laser may only be fired during the electron ionization of the target.

7. The system of claim 1, wherein the fractional optical system comprises a lens array for concentrating the laser emission to two or more adjacent targets at a depth in the tissue at a fluence sufficient to create electron ionization of the two or more adjacent targets.

8. The system of claim 1, wherein the laser is configured to avoid thermal damage in a region adjacent the targets in response to pulse width adjustments of the laser.

9. The system of claim 1, wherein the laser has a wavelength of about 755 nm.

10. The system of claim 1, wherein the laser has a wavelength of about 1064 nm.

**Patentansprüche**

1. System zur Gewebebehandlung, das Folgendes umfasst:

   einen Pikosekundenlaser, der zur Emission von Licht von mindestens einer Wellenlänge konfiguriert ist;
   ein fraktioniertes optisches System, das einen Linsen-Array umfasst, der eine Vielzahl von Brennpunkten zur Konzentration einer Laseremission von dem Pikosekundenlaser auf Ziele in der Epidermis einschließt, wobei die Ziele Licht von mindestens einer Wellenlänge absorbieren, und
   wobei der Pikosekundenlaser zur Emission einer Pulsbreite innerhalb des Bereichs von etwa 260 Pikosekunden bis etwa 900 Pikosekunden und eines Fluenzbereichs von etwa 0,8 J/cm$^2$ bis etwa 50 J/cm$^2$ konfiguriert ist, wobei die mindestens eine Wellenlänge von Melanin absorbiert wird und die Konzentration der Laseremission an den Zielen eine Elektronenlawinen-Ionisation an den Zielen auslöst, um eine Druckwellenemission auf Gewebe, das den Zielen benachbart ist, bereitzustellen.

2. System nach Anspruch 1, wobei die Ziele Melanin umfassen.

3. System nach Anspruch 1, wobei die Pulsbreite im Bereich von etwa 260 Pikosekunden bis etwa 500 Pikosekunden liegt.

4. System nach Anspruch 1, das weiter einen Regler zur Regelung der Pulsbreite umfasst.

5. System nach Anspruch 4, wobei der Regler die Pulsbreite regelt, um eine größere thermische Wirkung bei einer relativ längeren Pulsbreite bereitzustellen, oder wobei die Feineinstellung des Reglers auf eine andere Pulsbreite das Verhältnis von mechanischer zu thermischer Wirkung auf das Gewebe, das den Zielen benachbart ist, verändert.

6. System nach Anspruch 4, wobei der Regler eine kürzere Pulsbreite, die in einer relativ flachen Tiefe auf die Ziele konzentriert ist, und eine weitere relativ längere Pulsbreite in einer relativ tieferen Tiefe auf nachfolgende Ziele ermöglicht; und/oder wobei der Regler Folgendes ermöglicht: einen Durchgang des Lasers, wobei der mindestens eine Brennpunkt auf eine erste Tiefe konzentriert ist, und einen weiteren Durchgang des Lasers, wobei der mindestens eine Brennpunkt auf eine zweite Tiefe konzentriert ist, die sich von der ersten Tiefe unterscheidet; und/oder wobei der Regler einen ersten Puls des Lasers ermöglicht und ein zweiter Puls des Lasers nur während der Elektronenionisation des Ziels abgefeuert werden kann.

7. System nach Anspruch 1, wobei das fraktionierte optische System einen Linsen-Array für die Konzentration der Laseremission auf zwei oder mehr benachbarte Ziele in einer Tiefe in dem Gewebe bei einer Fluenz, die ausreicht, um Elektronenionisation der zwei oder mehr benachbarten Ziele zu erzeugen, umfasst.

8. System nach Anspruch 1, wobei der Laser konfiguriert ist, um thermischen Schaden in einem Bereich, der den Zielen benachbart ist, als Reaktion auf Pulsbreiteneinstellungen des Lasers zu vermeiden.

9. System nach Anspruch 1, wobei der Laser eine Wellenlänge von etwa 755 nm aufweist.

10. System nach Anspruch 1, wobei der Laser eine Wellenlänge von etwa 1064 nm aufweist.

**Revendications**

1. Système pour un traitement de tissu, comprenant :

   un laser picoseconde configuré pour émettre de la lumière d'au moins une longueur d'onde ;

un système optique fractionné comprenant un réseau de lentilles incluant une pluralité de foyers pour la concentration d'une émission laser, provenant du laser picoseconde, vers des cibles dans l'épiderme, où les cibles absorbent de la lumière d'au moins une longueur d'onde, et

le laser picoseconde configuré pour émettre une largeur d'impulsion au sein de la plage allant d'environ 260 picosecondes à environ 900 picosecondes et une plage de fluence allant d'environ 0,8 J/cm$^2$ à environ 50 J/cm$^2$, où l'au moins une longueur d'onde est absorbée par la mélanine et la concentration de l'émission laser au niveau des cibles initie une ionisation électronique par avalanche au niveau des cibles pour fournir une émission d'onde de pression au tissu adjacent aux cibles.

2. Système selon la revendication 1, où les cibles comprennent de la mélanine.

3. Système selon la revendication 1, où la largeur d'impulsion se situe dans une plage allant d'environ 260 picosecondes à environ 500 picosecondes.

4. Système selon la revendication 1, comprenant en outre un dispositif de commande pour la commande de la largeur d'impulsion.

5. Système selon la revendication 4, où le dispositif de commande commande la largeur d'impulsion pour fournir un effet thermique plus important à une largeur d'impulsion relativement plus longue ou où le réglage du dispositif de commande sur une largeur d'impulsion différente change le rapport de l'effet mécanique à l'effet thermique sur le tissu adjacent aux cibles.

6. Système selon la revendication 4, où le dispositif de commande permet une largeur d'impulsion plus courte concentrée à une profondeur relativement peu profonde vers les cibles et une autre largeur d'impulsion relativement plus longue à une profondeur relativement plus profonde vers les cibles suivantes ; et/ou

où le dispositif de commande permet un passage du laser avec l'au moins un foyer concentré à une première profondeur et un autre passage du laser avec l'au moins un foyer concentré à une deuxième profondeur différente de la première profondeur ; et/ou

où le dispositif de commande permet à une première impulsion du laser et à une deuxième impulsion du laser d'être déclenchées uniquement pendant l'ionisation électronique de la cible.

7. Système selon la revendication 1, où le système optique fractionné comprend un réseau de lentilles pour la concentration de l'émission laser sur deux cibles adjacentes ou plus à une profondeur dans le tissu à une fluence suffisante pour créer une ionisation électronique des deux cibles adjacentes ou plus.

8. Système selon la revendication 1, où le laser est configuré pour éviter des dommages thermiques dans une région adjacente aux cibles en réponse à des ajustements de largeur d'impulsion du laser.

9. Système selon la revendication 1, où le laser a une longueur d'onde d'environ 755 nm.

10. Système selon la revendication 1, où le laser a une longueur d'onde d'environ 1 064 nm.

Figure 1A

Figure 1B

Figure 2(a)

Figure 2(b)

330

315A    315B

-0.5mm-

-1.0mm-

-2.0mm-

Figure 3(a)

Figure 3(b)

Figure 3(c)

EP 2 969 369 B1

315　　　　　　　　　　　　　　　　　　　312
　　　　　　　　　　　　　　　　　　　　　　　330

-0.5mm-

-1.0mm-　　　　　　　　　　300

　　　　　　　　　　　　　　　　　　　301

-2.0mm-　　　　　　　　　　　　　　　306

## Figure 3(d)

315B　315A　315B　315A　　　　　　　330

-0.5mm-
　　　　320A　　　　320A
-1.0mm-
　320B　　　　320B　　　　　300

　　　　　　　　　　　　　　　301

-2.0mm-　　　　　　　　　　　　　306

## Figure 3(e)

Figure 3(f)

417A

417B

417C

420

417D

433

417E

417F

## Figure 4(a)

441

417A

417A

417B

445B

417B

443

445C

417C

417C

417D

417D

417E

417E

417F

417F

t LIOB first

distance

## Figure 4(b)

A

B

C

D

E

F

## Figure 4(c)

Figure 5(a)

Figure 5(b)

Figure 5(c)

Figure 5(d)

Figure 6

EP 2 969 369 B1

Figure 7(a)

Figure 7(b)

Figure 8(a)

Figure 8(b)

## REFERENCES CITED IN THE DESCRIPTION

### Patent documents cited in the description

- US 2008031288 A
- US 7929579 B
- US 7586957 B
- US 2011313408 A
- US 6997923 B
- US 7856985 B
- US 8322348 B
- US 8317779 B
- US 11347672 B
- US 12635295 B
- US 12947310 B
- US 10026432 W

### Non-patent literature cited in the description

- **HABBEMA et al.** *J. Biophotonics,* 2012, vol. 5 (2), 194-199
- **ORAEVSKY et al.** *IEEE Journal of Selected Topics in Quantum Electronics,* December 1996, vol. 2, 801-809